# EUROPEAN PATENT SPECIFICATION

(11) **EP 2 605 755 B1**
(45) Date of publication and mention of the grant of the patent: **31.07.2019**
(21) Application number: 11817565.2
(22) Date of filing: 15.08.2011
(51) Int. Cl.: A61K 9/51, A61K 9/14, A61K 38/00, A61K 48/00, A61K 31/70, A61K 47/02, C01B 33/18

(54) **PARTICULATE SUBSTANCES COMPRISING CERAMIC PARTICLES FOR DELIVERY OF BIOMOLECULES**
PARTIKULÄRE SUBSTANZEN MIT KERAMIKPARTIKELN ZUR ABGABE VON BIOMOLEKÜLEN
SUBSTANCES PARTICULAIRES COMPRENANT DES PARTICULES DE CÉRAMIQUE DESTINÉES À L'ADMINISTRATION DE BIOMOLÉCULES

(30) Priority: 16.08.2010 AU 2010903683
(43) Date of publication of application: 26.06.2013
(73) Proprietor: AUSTRALIAN NUCLEAR SCIENCE & TECHNOLOGY ORGANISATION, Lucas Heights New South Wales 2234 (AU)
(72) Inventor: BARBÉ, Christophe, Jean, Alexandre, Five Dock NSW 2046 (AU); FINNIE, Kim, Suzanne, Chatswood West NSW 2067 (AU); KNIGHT, Samuel, Mundaring W.A. 6073 (AU); PASSIOURA, Toby, Johnston, Surry Hills NSW 2010 (AU)
(74) Representative: Plougmann Vingtoft a/s
(86) International application number: PCT/AU2011/001040
(87) International publication number: WO 2012/021922

(56) References cited:
- EP-A1- 1 645 597
- WO-A1-2006/050579
- WO-A1-2006/133519
- WO-A1-2010/090596
- PENG J ET AL: "An antisense oligonucleotide carrier based on amino silica nanoparticles for antisense inhibition of cancer cells", NANOMEDICINE: NANOTECHNOLOGY, BIOLOGY AND MEDICINE, ELSEVIER, NL, vol. 2, no. 2, 1 June 2006 (2006-06-01), pages 113-120, XP028009002, ISSN: 1549-9634, DOI: 10.1016/J.NANO.2006.04.003 [retrieved on 2006-06-01]
- XIA T. ET AL.: 'Polyethyleneimine Coating Enhances the Cellular Uptake of Mesoporous Silica Nanoparticles and Allows Safe Delivery of siRNA and DNA Constructs' ACS NANO vol. 3, no. 10, 2009, pages 3273 - 3286, XP055077005
- KAPOOR S. ET AL.: 'Influence of Surface Chemistry of Mesoporous Alumina with Wide Pore Distribution on Controlled Drug Release' JOURNAL OF CONTROLLED RELEASE vol. 140, no. 1, 2009, pages 34 - 39, XP026691080
- YEZHELYEV M.V. ET AL.: 'Proton-Sponge Coated Quantum Dots for siRNA Delivery and Intracellular Imaging' JOURNAL OF THE AMERICAN CHEMICAL SOCIETY vol. 130, no. 28, 2008, pages 9006 - 9012, XP055077007
- HILLARD L R ET AL: "Immobilization of oligonucleotides onto silica nanoparticles for DNA hybridization studies", ANALYTICA CHIMICA ACTA, ELSEVIER, AMSTERDAM, NL, vol. 470, 1 January 2002 (2002-01-01), pages 51-56, ISSN: 0003-2670
- FINNIE KIM S ET AL: "Formation of silica nanoparticles in microemulsions.", LANGMUIR : THE ACS JOURNAL OF SURFACES AND COLLOIDS 13 MAR 2007, vol. 23, no. 6, 13 March 2007 (2007-03-13) , pages 3017-3024, ISSN: 0743-7463
- HE XIAOXIAO ET AL: "A novel gene carrier based on amino-modified silica nanoparticles", CHINESE SCIENCE BULLETIN, vol. 48, no. 3, March 2003 (2003-03), pages 223-228, ISSN: 2095-9273
- HE XIAOXIAO ET AL: "A NOVEL DNA-ENRICHMENT TECHNOLOGY BASED ON AMINO-MODIFIED FUNCTIONALIZED SILICA NANOPARTICLES", JOURNAL OF DISPERSION SCIENCE AND TECHNOL, TAYLOR AND FRANCIS GROUP, NEW YORK, NY, US, vol. 24, no. 3-4, 1 May 2003 (2003-05-01), pages 633-640, ISSN: 0193-2691, DOI: 10.1081/DIS-120021820
- CJA Barbé: "Declaration under 37 C.F.R. § 1.132", 12 July 2017 (2017-07-12)

## Description

### FIELD OF THE INVENTION

The present invention relates to particulate substances that comprise ceramic particles for delivery of biomolecules and to methods for making them. More particularly, the invention relates to particulate substances that comprise particles of a ceramic matrix bearing an aminofunctional group that have releasable biomolecules disposed within pores of the particles.

### BACKGROUND OF THE INVENTION

Use of siRNA and gene therapy represents a potential major advance in healthcare. It shows the potential to treat a range of currently non-curable diseases such as cystic fibrosis, some cancers, and immune disease such as Type 1 diabetes, multiple sclerosis etc. There is however a need to protect siRNA from enzymatic degradation in vivo until delivery to the site of action in order to provide effective therapy.

At present, siRNA therapy is expensive. The major markets for such expensive therapies are primarily in the more developed countries. The global market for gene therapy is estimated to be >US$5B.

Major challenges in developing siRNA therapy to clinical use include:
- protection of the active material from enzymatic degradation;
- enabling the active material to enter the target cells (good penetration);
- release of the active material from an encapsulant in the cytoplasm (endosomal escape);
- ensuring the ability to knock down genes (preferably with efficacy at nM concentration); and
- achievement of low toxicity (large therapeutic window).
siRNAs are intermediate sized (about 14kDa, 3nm diameter, 10nm length), hydrophilic, strongly negatively charged molecules. They are both chemically and biologically labile unless modified to enhance stability. In order to achieve a clinical effect, siRNAs must be able to cross the cellular membrane and be present in the cytoplasm of the target cell population.

In the past, viral vectors have been explored in order to deliver RNA or DNA. However these suffer from the risk of immunological reactions and are difficult to put into practice. Various non-viral vectors (e.g. lipid complexes, cationic polymer complexes, liposomes, dendrimers, polymeric nanoparticles) have also been explored. These provide a range of problems, including difficulty in implementation with siRNA, interactions between the siRNA and the vector, and exposing the siRNA to degradation *in vivo.* In particular, various systems have been devised for adsorbing DNA or RNA onto the surface of nanoparticles. However these generally suffer from the disadvantage that the adsorbed biomolecule is subject to enzymatic attack prior to delivery to the site of action, thereby reducing the effectiveness of treatment.

While the above discussion relates primarily to siRNA and DNA, the problems discussed are not limited to siRNA and DNA. They potentially apply to a wide range of biomolecules, including for example peptides, proteins and so on, for which intracellular delivery is desired. Thus a solution to these problems may be more widely applicable. The application of the invention should not therefore be considered limited to siRNA.

Peng et al, "An antisense oligonucleotide carrier based on amino silica nanoparticles for antisense inhibition of cancer cells", Nanomedicine, Biology and Medicine, Elsevier, NL, vol. 2, no. 2, 1 June 2006 (2006-06-01), pages 113-120, describes preparation of amino silica nanoparticles.

Advantageously, the present invention substantially overcomes or at least ameliorates one or more of the above disadvantages and at least partially satisfies the above need.

### SUMMARY OF THE INVENTION

According to one aspect of the invention there is provided a particulate substance comprising:
particles of a ceramic matrix bearing an aminofunctional group, the aminofunctional group being capable of promoting penetration of the particles into cells and being distributed homogeneously throughout said particles; and
a biomolecule disposed within pores of the particles, the biomolecule being releasable from the particles by dissolution of the ceramic matrix.

As used herein, reference to the biomolecule being "disposed within pores of the particles" is intended to include within its scope embodiments where the ceramic matrix, which effectively forms solid porous particles, has biomolecules dispersed throughout or disposed in the pores of the ceramic matrix. This is not intended to include situations where the biomolecule is attached or bound to the outer surface of the particles.

Generally, other than possibly under relatively extreme conditions, the biomolecule is substantially non-releasable from the particles by leaching in the absence of dissolution of the ceramic matrix. In that regard, as used herein, reference to the biomolecule being "substantially non-releasable by leaching in the absence of dissolution" is intended to include within its scope leaching under the proposed conditions of storage and use of the particulate substance. Preferably, the functional group interacts with the biomolecule to substantially prevent leaching.

According to one embodiment of the invention the ceramic matrix bearing an aminofunctional group comprises an aminofunctionalised silica matrix. However, a range of metal oxides including mixed metal oxides may be suitable, for example titania, alumina, zirconia, iron oxide, ceria, zinc oxide, and so on. The aminofunctional group may also be provided either by an organotitatnia or organo-alumina, or by an organo-silane that will co-condense with another metal precursor forming an organo titania silica or organo-alumino-silica. Further embodiments will be appreciated from the discussions relating to preparation of the particles which follow.

In a preferred embodiment the aminofunctional group comprises an aminoalkylamino group, a primary alkylamino group, a secondary alkylamino group, and tertiary alkylamino group, an alkylimidazole group, an alkylamide group or an alkylamino acid group. Further embodiments will be appreciated from the discussions relating to preparation of the particles which follow.

The present invention relates to a particulate substance comprising a biomolecule. In this context, the term "biomolecule" may refer to a substance of a biological origin or nature and having biological activity. The term includes within its scope a substance comprising one or more molecules including a mixture of different molecules. The biomolecule may be a macromolecule. It may have a molecular weight of about 1 to about 1000kDa or more, or about 1 to about 100, 1 to 50, 1 to 20, 1 to 10, 5 to 1000, 10 to 1000, 100 to 1000, 500 to 1000, 5 to 100, 5 to 50, 5 to 20 or 10 to 20kDa, e.g. about 1, 2, 3, 4, 5, 6, 7, 8, 9, 10, 11, 12, 13, 14, 15, 16, 17, 18, 19, 20, 30, 40, 50, 60, 70, 80, 90, 100, 200, 300, 400, 500, 600, 700, 800, 900 or 1000kDa. In some instances it may have molecular weight of less than 1kDa or greater than 1000kDa. It may have a diameter of about 0.5-20 nm, or about 1 to 20, 2 to 20, 5 to 20, 10 to 20, 0.5 to 10, 0.5 to 5, 0.5 to 2, 0.5 to 1, 1 to 10, 2 to 10, 1 to 5, 5 to 10 or 10 to 20nm, e.g. about 0.5, 1, 2, 3, 4, 5, 6, 7, 8, 9, 10, 15 or 20nm.

The biomolecule may be selected depending on the particular application in question. In order to achieve retention of the biomolecule in and/or on the particles, it may be negatively charged. This may enable the biomolecule to bind to an aminofunctional group on the ceramic matrix, e.g. to protonated amine groups in an aminofunctional ceramic matrix. Alternatively or additionally the biomolecule may have other functionality that enables it to bind to aminofunctional groups of the ceramic matrix. Alternatively or additionally the biomolecule may be sufficiently large (i.e. have a sufficiently large molecular weight or molecular volume) that it is physically trapped in the particles. It may be sufficiently large that it is incapable of passing through the pores of the particles.

In certain embodiments the biomolecule may be nucleic acid such as an RNA, for example an siRNA (small interfering RNA), miRNA (microRNA) or a ribozyme, an ASODN (antisense nucleotide or antisense RNA), a DNA molecule, an aptamer, a protein inclusive of polypeptides, peptides, glycoproteins, lipoproteins, immunoglobulins (e.g antibodies and antibody fragments), a carbohydrate, a lipid or a mixture or adduct of any two or more of these. In one particular embodiment the biomolecule is an siRNA. The biomolecule may be indicated for prophylactic or therapeutic treatment of a disease, disorder or condition.

It will be advantageous in some embodiments to bind a surface treating agent to the surface of the particles. In a preferred embodiment, polyethylene glycol (PEG) chains are coupled to the surface of the particles. Alternatively or additionally, a targeting group may be coupled to the surface of the particles to facilitate targeting of the particles to a target, for example a tumour or particular organ or other target, in use. In certain embodiments, PEG chains having targeting groups at their distal ends may be coupled to the particles.

In certain embodiments, it may be preferred that the particles of the particulate substance have a mean particle size of about 0.1 to about 1 micron. However, they may have a mean particle size of about 0.1 to 10 microns, or about 0.1 to 5, 0.1 to 2, 0.1 to 1, 0.1 to 0.5, 0.2 to 10, 0.5 to 10, 1 to 10, 2 to 10, 5 to 10, 0.2 to 2, 0.2 to 1, 0.2 to 0.5, 0.5 to 2 or 0.5 to 1 micron, e.g. about 0.1, 0.2, 0.3, 0.4, 0.5, 0.6, 0.7, 0.8, 0.9, 1, 1.5, 2, 2.5, 3, 3.5, 4, 4.5, 5, 6, 7, 8, 9 or 10 microns.

In some embodiments, it may be preferred that the mean particle size be less than about 0.1 microns. For example, it may be about 20 to 100nm (0.1 micron), or about 20 to 50nm, or about 50 to 100nm, e.g. about 20, 30, 40, 50, 60, 70, 80 or 90nm.

It is noted that particles above about 1-2 microns in size may be unsuitable for intracellular delivery. However, it is considered that they may be useful for delivery of larger proteins elsewhere in the body. In that regard, particles up to several microns may be internalised, particularly by specialised phagocytotic cells.

The particles may be substantially monodispersed or there may be some aggregation to form a second peak in the particle size distribution curve. The distribution curve may be normal, Gaussian or some other distribution. The particles may have a broad particle size distribution or a medium or narrow particle size distribution. The particles may be spherical, or approximately spherical, or may be ovoid or oblate spherical or polyhedral (having e.g. 8 to about 60 sides) or may be some other shape. They may be irregular in shape.

The particles may be mesoporous (i.e. <100nm pore size). They may be microporous (i.e. <1.7nm pore size). Preferably, the particles have a mean pore size of about 1 to about 50nm. For example, the mean pore size may be about 1 to 20, 1 to 10, 5 to 50, 10 to 50, 20 to 50, 5 to 20, 5 to 10 or 10 to 20nm, e.g. about 1, 2, 3, 4, 5, 10, 15, 20, 25, 30, 35, 40, 45 or 50nm.

The pore structure may comprise interconnected pores, or may comprise voids joined by relatively small interconnecting channels. The pore size may be sufficiently small so as to substantially prevent release of the biomolecule by diffusion from the pores. Alternatively, if the pore size is such that the biomolecule can escape, the biomolecule may be retained by attraction to functional groups on the pore surfaces. The functional groups may be the same or different to those which promote penetration of the particles into cells.

The particles may have a loading of biomolecule from about 1 to about 20% w/w, for example about 1 to 10, 1 to 5, 1 to 2, 2 to 20, 5 to 20, 10 to 20, 2 to 10, 2 to 5 or 5 to 10%, e.g. about 1, 2, 3, 4, 5, 6, 7, 8, 9, 10, 11, 12, 13, 14, 15, 16, 17, 18, 19 or 20%, although in some cases it may be less than 1% or may be greater than 20%.

In use, the biomolecule is advantageously releasable by dissolution of the particles under conditions which do not substantially degrade the biomolecule. For example, it may be releasable by dissolution of the particles by a biological medium which does not substantially degrade the biomolecule. It may alternatively be releasable when diluted in a suitable release liquid.

Generally, the biomolecule is releasable (e.g. substantially completely releasable) over a period of about 0.5 to about 50 hours. For example, about 0.5 to 20, 0.5 to 10, 0.5 to 5, 0.5 to 2, 1 to 50, 5 to 50, 10 to 50, 1 to 20, 1 to 10, 2 to 10 or 5 to 10 hours, e.g. about 0.5, 1, 2, 3, 4, 5, 6, 7, 8, 9, 10, 15, 20, 25, 30, 35, 40, 45 or 50 hours. As the rate of dissolution may be dependent on the size of the particles, this rate may be adjusted by adjusting the size of the particles as discussed in the following description.

Most advantageously, the biomolecule is protected from degradation prior to its release from the particles when the particles are exposed to a degradation agent, e.g. an enzyme, which would otherwise be capable of degrading the biomolecule. That is, the biomolecule is protected from degradation by the ceramic matrix.

In some embodiments, it is envisaged that a polymer or complexing agent may be disposed in the pores of the particles with the biomolecule to facilitate endosomal escape. Typically the polymer could be a polyethylinamine, a polylysine, or a polyhistidine or any substance that provides a proton sponge effect.

### Formation of micro-particles (>100nm)

In some embodiments, it may be advantageous to form particles on the micro scale. That is, for the purpose of this description, particles of greater than 100nm in mean particle size.

According to another aspect of the invention there is provided a process for making particles comprising a biomolecule dispersed in pores thereof, said process comprising:
a) combining:
   a hydrophobic phase comprising a hydrophobic liquid, a first ceramic precursor and a surfactant; and
   a hydrophilic phase comprising a hydrophilic liquid, a second ceramic precursor and the biomolecule,
   so as to form an emulsion comprising droplets of the hydrophilic phase dispersed in the hydrophobic phase; and
b) agitating the emulsion as the particles form inside the droplets;
wherein the first ceramic precursor comprises an aminofunctional group which is capable of promoting penetration of the particles into cells.

As used herein the term "agitating" includes within its scope any form of agitation, including but not necessarily limited to stirring, shaking, swirling, sonicating, shearing and so on, and any combination of these.

In making the particles, an emulsion is formed by combining a hydrophobic phase with a hydrophilic phase. This may be a water-in-oil (w/o) emulsion, in which the hydrophobic phase represents the continuous phase and the hydrophilic phase represents the dispersed or discontinuous phase.

The hydrophobic phase may be an oleophilic phase or a lipophilic phase. The hydrophobic phase may be made by combining the surfactant with the hydrophobic liquid and adding the first ceramic precursor so as to form the hydrophobic phase, or it may be made by combining all three components, or it may be made by combining the first ceramic precursor with either the hydrophobic liquid or the surfactant and then adding the other. These steps are preferably conducted prior to combining the hydrophobic and hydrophilic phases. Each combining step may comprise agitating the components which have been combined. The agitation may comprise stirring, shaking, swirling, sonicating or a combination of these. It may be sufficient for the components to form a solution. Thus the hydrophobic phase may represent a solution of the first ceramic precursor and the surfactant in the hydrophobic liquid.

The hydrophobic phase comprises 3 components:
Hydrophobic liquid - this may be, for example, a vegetable oil, paraffin oil, mineral oil or some other suitable hydrophobic liquid. It may comprise a mixture of hydrophobic components, e.g. a mixture of vegetable oils or a mixture of vegetable oil and paraffin oil. It is commonly of moderate viscosity, e.g. about 0.5 to about 1500mPa.s, or about 0.5 to 1000, 0.5 to 500. 0.5 to 250, 0.5 to 100, 0.5 to 50, 0.5 to 20, 0.5 to 10, 0.5 to 5, 0.5 to 1, 1 to 1500, 10 to 1500, 100 to 1500, 250 to 1500, 500 to 1500, 1000 to 1500, 10 to 1000, 10 to 200, 200 to 1000 or 200 to 500mPa.s, e.g. about 0.5, 1, 2, 3, 4, 5, 6, 7, 8, 9, 10, 15, 20, 25, 30, 35, 40, 45, 50, 60, 70, 80, 90,100,150, 200, 250, 300, 350, 400, 450, 500, 600, 700, 800, 900, 1000, 1100, 1200, 1300, 1400 or 1500mPa.s, or, on some occasions, greater than 1500mPa.s. The viscosity of the hydrophobic liquid may be used in order to control the particle size of the particles produced by the process. Thus a more viscous hydrophobic liquid will generally provide a more viscous hydrophobic phase, which in turn will generally provide a smaller particle size.

Surfactant - this may be a suitable surfactant for supporting a water-in-oil emulsion. It may be soluble in, or miscible with, the hydrophobic liquid. It may be a non-ionic surfactant or it may be an anionic surfactant or it may be a zwitterionic surfactant. It may have an HLB of about 8 to about 16, or about 8 to 12, 10 to 16 or 8 to 10, e.g. about 8, 9, 10, 11, 12, 13, 14, 15 or 16. Suitable surfactants include Span® 20 (Sorbitan monolaurate), Aerosol® OT (sodium bis(2-ethylhexyl) sulfosuccinate), Span® 20/Tween® 80 mixtures and Span® 20/Brij® 35 mixtures. Use of the mixed surfactants commonly provides a very fine emulsion, but the final particle size is generally unchanged.

First ceramic precursor - This component includes an aminofunctional group capable of promoting penetration of the resulting particles into cells. In certain embodiments, the aminofunctional group of the first ceramic precursor is capable of chemically interacting with, for example electrostatically interacting with, the biomolecule.

This component may be a compound having at least one amine group per molecule and being capable of being converted into an aminofunctional ceramic matrix. It may be soluble in the hydrophobic liquid, or in a mixture (optionally a solution) of the surfactant in the hydrophobic liquid.

Suitable ceramic precursors include aminofunctional silanes, in particular aminofunctional alkoxysilanes. The alkoxy groups of these silanes may be for example C1 to C6 alkoxy groups (which may be branched if C3 or greater), commonly C1 to C4 alkoxy, e.g. methoxy, ethoxy, propoxy, isopropoxy or butoxy groups. In some cases other hydrolysable groups may be used, e.g. acetoxy, ketoximo, enoloxy etc. The aminofunctional ceramic precursor may have more than one amine group per molecule, e.g. 2, 3,4 or 5 amine groups per molecule. The inventors have found that diamino- and triamino-ceramic precursors commonly produce particles which are more effective at binding suitable biomolecules than the corresponding monoamino-ceramic precursors. Each amine group may, independently, be primary, secondary or tertiary. In preferred precursors, the amine groups are separated by linker groups, commonly short alkylene chains such as ethylene (-CH₂CH₂-), propylene (-CH₂CH₂CH₂-), or butylene (-CH2CH2CH2CH2-) chains. The inventors consider that the butylene group may be particularly useful because this group occurs in naturally occurring polyamine polynucleotide ligands such as putrescine (N-4-N), spermidine (N-3-N-4-N) and spermine (N-3-N-4-N-3-N). Various combinations involving pentylene and hexylene may also be useful, however groups that are too different to the biogenic configuration may be potentially toxic. In particular, the inventors consider that ethylene spacers provide a distance between the amine groups that is acceptably close to the spacings of charges in siRNA and is present in commercially available products, making this spacer suitable for use when the biomolecule is an siRNA. Thus suitable precursors include 3-(2-aminoethylamino)propyl trimethoxysilane, 3-[2-(2-aminoethylamino)ethylamino]propyl trimethoxysilane, 3-(2-aminoethylamino)propyl triethoxysilane or 3-[2-(2-aminoethylamino)ethylamino]propyl triethoxysilane and mixtures of any two or more thereof.

In some cases the first ceramic precursor may be a mixture. It may be a mixture of silane ceramic precursors. It may additionally comprise one or more non-silane ceramic precursors, for example a zirconia precursor, an alumina precursor, or a titania precursor. These may be for example zirconium alkoxides, aluminium alkoxides and titanium alkoxides respectively.

Commonly the ratio of surfactant to hydrophobic liquid is about 5 to about 25% w/v (i.e. about 5 to about 25 g surfactant to 100ml hydrophobic liquid) or about 5 to 20, 5 to 15, 10 to 25, 15 to 25 or 10 to 20%, e.g. about 5, 10, 15, 20 or 25%.

Commonly the ratio of the first ceramic precursor to hydrophobic liquid is about 10 to about 1000 ppm on a v/v basis, or about 10 to 500, 10 to 200, 10 to 100, 10 to 50, 20 to 1000, 50 to 1000, 100 to 1000, 200 to 1000, 500 to 1000, 20 to 500, 50 to 500, 50 to 200, 200 to 500 or 50 to 200ppm, e.g. about 10, 20, 304, 50, 60, 70, 80, 90,100,150, 200, 250, 300, 350,400, 450, 500, 600, 700, 800, 900 or 1000ppm.

The hydrophilic phase may be a lipophobic phase. It may be an aqueous phase. The hydrophilic phase comprises three components:
Hydrophilic liquid - this may be lipophobic. It is commonly aqueous, for example it may be water, including pure water, or an aqueous solution. It may also comprise dissolved salts.

Second ceramic precursor - this may be a water soluble silicate, particularly metasilicate. It may be silicate itself (e.g. by hydrolysis of a tetraalkylsilicate such as tetramethylorthosilicate or tetraethylorthosilicate), or may be a species with formula RSi(OR')ₓOH_{y}Si_{z} where x+y+z=3 (referred to herein as an alkylsilicate, generated for example by hydrolysis of an alkyltrialkoxysilane, e.g. methyltrimethoxysilane or ethyltrimethoxysilane). In the case of an alkylsilicate, the alkyl group R should be sufficiently small or sufficiently hydrophilic that the second ceramic precursor is water soluble. It will be understood that this may be achieved for example with small R groups such as methyl or ethyl, or with larger R groups having hydrophilic or polar substituents such as hydroxyl, nitro, sulphate, etc.

The second ceramic precursor may be, for example, waterglass. Waterglass is an oligomeric or polymeric silicate material having empirical formula about Na₂SiO₃, with varying degrees of hydration, commonly in aqueous solution. The waterglass may have a solids content of about 1 to about 20%, or about 1 to 10, 1 to 5, 1 to 2, 2 to 20, 5 to 20, 10 to 20, 2 to 10, 2 to 5 or 5 to 10%, e.g. about 1, 2, 3, 4, 5, 6, 7, 8, 9, 10, 11, 12, 13, 14, 15, 16, 17, 18, 19 or 20%. This may have about 25 to about 30% silica and about 1 to about 20% sodium hydroxide in water. It may be diluted by a factor of about 1:2 to about 1:10 in water, or about 1:2 to 1:5, 1:5 to 1:10 or 1:3 to 1:8, e.g. about 1:2, 1:3, 1:4, 1:5, 1:6, 1:7, 1:8, 1:9 or 1:10.

The second ceramic precursor may also be a titanium alkoxide (e.g. ethoxide, n and iso propoxide, n, sec and tert butoxide) or an aluminium alkoxide or a zirconium alkoxide or a modified metal alkoxide (e.g. modified with acetyl acetone or acetic acid). It could also be a mixed metal alkoxide. It could also be another metal salt like magnesium salt, zirconiuim salt, or aluminium salt to form magnesium silicate, alumino-silicate and so on. It may be a prehydrolised silicon alkoxide.

The second ceramic precursor may comprise a ceramic colloid, for example colloidal silica. The ceramic colloid may have a particle diameter below 50nm, or below about 40, 30, 20 or 10nm, or from about 5 to about 50nm or from about 5 to 20, 5 to 10, 10 to 50, 20 to 50 or 10 to 20nm. It may have a particle diameter (commonly mean particle diameter but optionally maximum particle diameter) of about 5, 10, 15, 20, 25, 30, 35, 40, 45 or 50nm.

In some cases the second ceramic precursor may comprise a combination of two or more of the above options, e.g. it may comprise a mixture of a water silicate with colloidal silica.

Biomolecule - various options for the biomolecule are recited above. As noted, this may be negatively charged or may be neutrally charged. It may be sufficiently negatively charged to be attracted to, optionally bound to, the functional group of the particles (derived from the first ceramic precursor). It may be, or may comprise, an RNA, e.g. an siRNA (small interfering RNA), miRNA (microRNA), ASODN (antisense nucleotide or antisense RNA), an aptamer, a DNA, a protein, a glycoprotein, a polypeptide, a carbohydrate or a mixture or adduct of any two or more of these.

Additionally a polymer or complexing agent could be added such that it is disposed within the pores of the particles with the biomolecule to facilitate endosomal escape. Typically the polymer could be a polyethylinamine, a polylysine, or a polyhistidine or any substance that provides a proton sponge effect.

The hydrophilic phase may be acidic. It may have a pH below the pKₐ of the first ceramic precursor (or of its conjugate acid if the ceramic precursor is a base, e.g. an aminofunctional ceramic precursor). The hydrophilic phase may have a pH less than about 10.5, or less than about 10, 9, 8, 7, 6, 5.5, 5, 4.5 or 4, or between about 3 and 10.5, 5 and 10.5, 7 and 10.5, 9 and 10.5, 7 and 10, 9 and 4, 7 and 4, 9 and 7, 5 and 7, 3 and 6, or about 3 to 5, 3 to 4, 4 to 6, 4 to 5 or 3.5 to 4.5, e.g. about 3, 3.5, 4, 4.5, 5, 5.5 or 6.

Commonly in preparing the hydrophilic phase the hydrophilic liquid and the second ceramic precursor are combined, optionally the second ceramic precursor is dissolved in the hydrophilic liquid. The process may subsequently comprise adjusting the pH to a pH below the pKₐ of the first ceramic precursor, for example a pH less than about 10.5, or to an acidic pH, for example to a pH less than about 7, or less than about 5, or less than about 4, and adding the biomolecule so as to form the hydrophilic phase. For example, in the event that the second ceramic precursor is waterglass or colloidal silica, this commonly results in a basic solution. The process may therefore comprise acidifying this solution.

Acidification may be conveniently achieved by exposing the solution to a cation exchange resin wherein, before said exposing, the resin is in its acid (protonated) form. The exposing may comprise combining the resin and the solution, optionally agitating the resulting mixture, and then separating the resin from the acidified solution (e.g. by filtration, decanting, centrifugation etc.), or it may comprise passing the solution through a bed of the resin. The ratio of resin to second ceramic precursor may be such that the desired pH (as described above) is achieved. Alternatively the second ceramic precursor may be acidified by addition of an acidifying agent (e.g. an acid) or of a suitable buffer.

Commonly the biomolecule will be added to the acidified solution shortly before the hydrophilic phase and the hydrophobic phase are combined. It may be added immediately before they are combined. It may be added less than about 2 minutes before they are combined, or less than about 1 minute, or less than about 50, 40, 30, 20, 15 or 10 seconds before they are combined, e.g. about 5, 10, 15, 20, 25, 30, 40, 50, 60, 70, 80, 90, 100, 110 or 120 seconds before they are combined. This reduces the possibility of adverse chemical reactions of the biomolecule occurring. The biomolecule may be present in the hydrophilic phase in sufficient quantity to achieve the desired loading in the final particles. A typical concentration of biomolecule in the hydrophilic phase is about 1 to about 10mg/ml, or about 1 to 5, 5 to 10 or 2 to 8, e.g. about 1, 2, 3, 4, 5, 6, 7, 8, 9 or 10mg/ml. The biomolecule may be added to the combined hydrophilic liquid/second ceramic precursor in the form of a solution. The solvent for this solution should be miscible with the hydrophilic liquid, and is commonly the same as the hydrophilic liquid. The biomolecule may be added in aqueous solution.

In forming the emulsion, the hydrophobic and hydrophilic phases are combined, optionally with agitation. The agitation may comprise one or more of stirring, shaking, swirling and sonicating. An effective way to make the emulsion is to prepare the hydrophobic phase as described above and subject it to simultaneous stirring and sonicating in preparation for addition of the hydrophilic phase. The hydrophilic phase is then prepared by combining the biomolecule with the combined second ceramic precursor and hydrophilic liquid (e.g. acidified aqueous waterglass solution), and the resulting hydrophilic phase is added as quickly as practicable to the sonicated, stirred hydrophobic phase while maintaining the sonication. Sonication may be continued for a short time following the addition, e.g. about 10 to about 120 seconds, or about 10 to 60, 10 to 30, 20 to 120, 60 to 120, 20 to 60 or 20 to 40 seconds, e.g. about 10, 20, 30, 40, 50, 60, 90 or 120 seconds. The sonicating is commonly turned off after a suitable period so as to prevent overheating of the emulsion. Such overheating could for example adversely affect the biomolecule. Sonication may be conducted at a power of about 200 to 2000W, or about 200 to 1000, 200 to 500, 500 to 2000, 1000 to 2000, 500 to 1000 or 600 to 800W, e.g. about 200, 300, 400, 500, 600, 700, 800, 900, 1000, 1200, 1400, 1600, 1800 or 2000W.

The ratio of hydrophobic phase to hydrophilic phase may be about 10 to about 50 (i.e. about 10:1 to about 50:1), or about 10 to 40, 10 to 30, 10 to 20, 20 to 50, 30 to 50, 40 to 50, 20 to 40 or 25 to 25, e.g. about 10, 15, 20, 25, 30, 35, 40, 45 or 50.

It may be sufficient for the molar ratio of first ceramic precursor to second ceramic precursor to be about 0.2 to about 20 mol%, or about 0.5 to 20, 1 to 20, 2 to 20, 5 to 20, 10 to 20, 0.2 to 10, 0.2 to 5, 0.2 to 2, 0.2 to 1, 1 to 10, 1 to 5 or 5 to 10, e.g. about 0.5, 1, 1.5, 2, 2.5, 3, 3.5, 4, 4.5, 5, 6, 7, 8, 9, 10, 11, 12, 13, 14, 15, 16, 17, 18, 19 or 20 mol%. The ratio of first ceramic precursor to second ceramic precursor may be varied in order to vary the charge on the particles. Thus if the amount is low (e.g. about 1 mol% relative to second ceramic precursor), the particles will be approximately neutral charge, whereas if the amount is higher (around 10 mol%) they will be positively charged.

In the emulsion prepared as described above, the droplets of the hydrophilic phase may have a mean diameter of about 0.1 to about 10 microns, or about 0.1 to 5, 0.1 to 2, 0.1 to 1, 0.1 to 0.5, 0.2 to 10, 0.5 to 10, 1 to 10, 2 to 10, 5 to 10, 0.2 to 2, 0.2 to 1, 0.2 to 0.5, 0.5 to 2 or 0.5 to 1 micron, e.g. about 0.1, 0.2, 0.3, 0.4, 0.5, 0.6, 0.7, 0.8, 0.9, 1, 1.5, 2, 2.5, 3, 3.5, 4, 4.5, 5, 6, 7, 8, 9 or 10 microns. The mean may be a number average or a weight average diameter. The droplets may be substantially monodispersed or there may be some aggregation to form a second peak in the distribution curve. The droplets may have a broad particle size distribution or a medium or narrow particle size distribution.

It is thought that the particles form inside the droplets by interaction of the first and second ceramic precursors. The condensation of the precursors to form the particles is commonly very rapid (milliseconds to seconds). The interaction may be a reaction. It may be a condensation. It may comprise hydrolysis of the first ceramic precursor. It has been observed that when the first ceramic precursor is added to the hydrophilic phase directly, rapid gelation occurs so that formation of suitably sized particles is prevented.

The combined hydrophilic and hydrophobic phases may be stirred or otherwise agitated for sufficient time for formation of the particles. This may depend at least in part on the temperature of the reaction. The particle formation may be conducted at any suitable temperature, e.g. room temperature, or about 10 to about 35°C, or about 10 to 30, 10 to 25, 10 to 20, 15 to 35, 20 to 35, 25 to 35, 15 to 30, 15 to 20 or 20 to 25°C, e.g. about 15, 20, 25, 30 or 35°C. It may be conducted at a temperature below the denaturation temperature of the biomolecule. The formation of the particles may take about 10 to about 120 minutes, although the combined phases may be stirred or otherwise agitated for longer than this if desired. Suitable times are about 10 to 100, 10 to 60, 10 to 30, 20 to 120, 30 to 120, 60 to 120, 30 to 90 or 45 to 75 minutes, e.g. about 10, 15, 20, 25, 30, 35, 40, 45, 50, 55, 60, 65, 70, 75, 80, 85, 90, 95, 100, 105, 110, 115 or 120 minutes.

Once the particles have formed, they may be surface functionalised. This may be achieved *in situ,* i.e. without separation or isolation of the particles. It may comprise adding a surface treating agent to the emulsion following formation of the particles so as to surface treat the particles. The surface functionalisation may be a PEGylation (i.e. adding polyethylene glycol chains to the surface). The surface treating agent may comprise a polyethylene glycol (PEG) chain coupled to a binding group. The PEG chain may have a molecular weight of about 1 to about 20kDa, or about 1 to 10, 1 to 5, 1 to 2, 2 to 20, 5 to 20, 10 to 20, 2 to 10, 2 to 5 or 5 to 10kDa, e.g. about 1, 2, 3, 4, 5, 6, 7, 8, 9, 10, 11, 12, 13, 14, 15, 16, 17, 18, 19 or 20kDa. The binding group may be a trialkoxysilane, i.e. the surface treating agent may be a trialkoxysilyl PEG. Suitable alkoxy groups include methoxy, ethoxy or propoxy. Other hydrolysable silyl groups may also be used, e.g. triacetoxy, trioximo, trienoloxy, triamido etc. The surface of the particles may be functionalised by reacting the surface with a PEG (or other suitable molecule) having a functional group that reacts either with the OH of the surface or amino groups incorporated inside and at the surface of the particles. For example a carboxyl functional PEG surface treating agent may be used to form an amide with surface amine groups on the particles, or the surface amine groups may be activated (e.g. by formation of succinimidyl groups or isothiocyanate groups) and then reacted with aminofunctional PEG surface treating agents.

The PEG groups are generally large (typically >1KDa) so that they will not penetrate inside the sphere but rather will graft primarily onto the surface of the particles. A range of functional PEGs is commercially available which are suitable for this grafting, for example isothiocyanate-modified PEG and carboxy-modified PEG, which will both produce amide bonds when reacted with amines on the surface of the particles.

Subsequent surface functionalisation, e.g. PEGylation, in order to functionalise the surface of the particles, may be limited but adequate in basic conditions. In basic conditions the first ceramic precursor (e.g. an aminosilane such as aminoethylaminopropyltriethoxysilane) may in some cases be added directly to the second ceramic precursor (e.g. waterglass or colloidal silica). In some cases the pH of the hydrophilic phase is not below the pKₐ of the first ceramic precursor. In such cases, the initially formed suspension of particles (made under basic conditions) may be subsequently acidified. This may serve to promote attachment of the biomolecule to the particles if the biomolecule is negatively charged. If the particles are subsequently surface treated, the acidification may be conducted before or during subsequent surface treatment so as to facilitate PEG-silane attachment. Without the presence of positive charges on the particles, release of the biomolecule may be very rapid (of the order of minutes) unless it is sufficiently large to prevent its escape through the pores of the particles.

In some cases the surface treating agent may comprise a targeting group for targeting a target in a patient. For example, the surface treating agent may comprise a trialkoxysilyl-PEG having the targeting group at the distal end of the PEG, i.e. it may have the structure trialkoxysilyl-PEG-targeting group. The target may be for example a tumour or a particular organ or some other target. The targeting group may for example be an antibody or an antibody fragment (e.g. an F_{ab}). Examples of suitable targeting groups include antibodies, peptide cytokines, peptide hormones, matrix proteins, cell-surface receptors, proteins involved in cell adhesion, proteins involved in cell recognition, proteins involved in cell motility, proteins involved in cell recruitment, proteins involved in cell differentiation, proteins involved in disease recognition, biologically active carbohydrates such as heparin and related substances, biologically active glycoproteins including but not limited to those which fall within the classes listed above, ligands of any member of the above classes, fragments of any member of the above classes, homologues of any member of the above classes, low-molecular-weight substances sharing the affinity or function of any member of the above classes, other low molecular weight biomolecules such as hormones, nutrients, drugs, toxins, neurotransmitters, endocrine transmitters, autocrine and paracrine transmitters, pigments, lipids, oils, ion ligands, metabolites, catabolites, etc.

The surface treating agent may be added directly to the suspension of particles in the hydrophobic phase. Reaction may be conducted suitably at around ambient temperature, e.g. by stirring for a suitable time to achieve reaction. Suitable times are about 8 to about 24 hours, or about 8 to 16, 8 to 12, 12 to 24, 18 to 24 or 12 to 18 hours, e.g. about 8, 12, 16, 20 or 24 hours. Sufficient surface treating agent may be used to achieve a suitable level of surface functionalisation, e.g. sufficient to prevent excessive particle aggregation or sufficient to provide acceptable targeting of the particles to the target in use.

Once the particles have been formed, it is common that they are not completely dried. This inhibits aggregation of the particles, which, if it occurred, would require resuspension which can in some circumstances be difficult. Commonly the particles are separated from the solution by centrifugation. Suitable conditions are about 10000 to about 50000rpm, or about 10000 to 30000, 30000 to 50000 or 20000 to 30000rpm, e.g. about 10000, 20000, 30000, 40000 or 50000rpm. Suitable separation is commonly achieved in about 5 to 15 minutes, although longer centrifugation may at times be used.

The resulting particles may be washed in order to remove impurities. The process of washing may involve resuspending the particles in a solvent, allowing the particles to at least partially separate from the solvent (e.g. by settling and/or by centrifugation) and decanting the solvent from the particles. It is important that the solvent is one that does not denature the biomolecule. This may be specific to the particular biomolecule used. For example ethanol does not affect the structure of DNA or RNA but may denature most large proteins. Suitable solvents for washing include hydrocarbons such as hexane, cyclohexane, toluene etc. and alcohols such as ethanol or isopropanol. The particles may be washed several times (e.g. 2, 3, 4 or 5 or more times), either with the same solvent or with different solvents.

The resulting particles may be resuspended in a suitable solvent and stored as a suspension in that solvent for later use. This solvent may be a clinically acceptable solvent if the particles are to be delivered to a patient. A suitable solvent for storage is ethanol. Commonly the particles will be stored at a temperature of about -210 to about +10°C, or about -210 to 0, -90 to 0, - 210 to -100, -210 to -65, -90 to -30, -30 to 0, -30 to -10, -20 to +10, -10 to +10, 0 to 10 or 0 to 5°C, e.g. about -210, -200, -180, -160, -140, -120, -100, - 90, -80, -70, -60, -50, -40, -30, -25, -20, -15, -10, -5, 0, 1, 2, 3, 4, 5, 6, 7, 8, 9 or 10°C. They may be stored at about liquid nitrogen temperature. They may be stored at dry ice temperature. They may be stored in a freezer or in a refrigerator.

In the above passage, where reference is made to ethanol, the ethanol may comprise up to about 30% water. Thus the ethanol may be about 70 to about 100% ethanol, the remainder being water, or about 80 to 100, 90 to 100, 70 to 90 or 80 to 90%, e.g. about 70, 80, 90 or 100% ethanol. Isopropanol, n-propanol or n-butanol may also be substituted for ethanol, with similar restrictions on water content. An advantage of the use of ethanol or propanol is that it provides a sterile environment for the particles for delivery to a patient or for other applications in which sterility is a benefit. In some instances methanol may be used.

The encapsulation efficiency (EE) of the process with regard to the biomolecule is preferably high, as the biomolecule is typically expensive. The EE will depend on the precise nature of the process, including for example the type and amount of first ceramic precursor, the ratio of biomolecule to ceramic precursors used etc. Commonly the process will deliver EE of greater than about 40%, or greater than about 50, 60, 70 or 80%. The EE may be for example about 40, 45, 50, 55, 60, 65, 70, 75, 80, 85, 90 or 95%.

In a particular embodiment there is provided a process for making particles comprising a biomolecule, the process comprising:
a) combining:
   a hydrophobic phase comprising a hydrophobic liquid, an aminoalkylaminofunctional trialkoxysilane and a surfactant having HLB between about 8 and about 16; and
   a hydrophilic phase comprising water, waterglass at about pH 5 and the biomolecule,
   so as to form an emulsion comprising droplets of the hydrophilic phase dispersed in the hydrophobic phase; and
b) agitating the emulsion as the particles form from the droplets.

In another particular embodiment there is provided a process for making particles comprising a biomolecule, the process comprising:
combining a surfactant having HLB between about 8 and about 16 with a hydrophobic liquid and adding an aminoalkylaminofunctional trialkoxysilane so as to form a hydrophobic phase;
combining water and waterglass, adjusting the pH to less than about 5 and adding the biomolecule so as to form a hydrophilic phase;
combining the hydrophobic phase and the hydrophilic phase so as to form an emulsion comprising droplets of the hydrophilic phase dispersed in the hydrophobic phase;
agitating the emulsion as the particles form from the droplets; and
adding a surface treating agent to the emulsion following formation of the particles so as to surface treat the particles.

In this embodiment, the step of adding the biomolecule may be conducted immediately (e.g. less than 1 minute) prior to the step of combining the hydrophobic and hydrophilic phases. The biomolecule may be an RNA or a DNA or other biomolecule as described previously. It may be an siRNA.

In variants of the above embodiment, other pH ranges may be used instead of less than about 5. For example basic conditions such as pH greater than about 8 may be used. The inventors consider that pHs in the range of about 5 to about 8 would also be usable. Other possible variants include use of a colloidal suspension, such as colloidal silica, in place of the water/waterglass combination. These variants may be suitable for encapsulation of relatively large biomolecules such as proteins.

### Formation of nano-particles (<100nm)

In various embodiments it may be advantageous to form particles on a smaller scale, particularly of less than 100nm in size. It is envisaged that this may provide for more effective delivery of the biomolecule in some instances.

Accordingly, the invention also provides a process for making particles comprising a biomolecule disposed in pores thereof, the process comprising:
a) combining:
   a hydrophobic phase comprising a hydrophobic liquid and a surfactant; and
   a hydrophilic phase comprising a hydrophilic liquid comprising water and a catalyst comprising an acid,
   so as to form an emulsion comprising droplets of the hydrophilic phase dispersed in the hydrophobic phase;
b) adding a ceramic precursor to the emulsion and hydrolysing the ceramic precursor;
c) adjusting the pH of the hydrophilic phase to a range suitable for the biomolecule;
d) adding the biomolecule and a functionalised ceramic precursor to the emulsion; and
e) agitating the emulsion as the particles form inside the droplets,
wherein the functionalised ceramic precursor comprises an aminofunctional group which is capable of promoting penetration of the particles into cells.

Many of the features and embodiments discussed above in relation to the preparation of micro-particles may equally apply to this aspect of the invention. As such, these features and embodiments are explicitly incorporated herein by reference in order to avoid unnecessary repetition. In that regard, the functionalised ceramic precursor described in accordance with this aspect of the invention corresponds with the first ceramic precursor discussed above. The ceramic precursor described in accordance with this aspect of the invention corresponds with the second ceramic precursor discussed above.

Notwithstanding the incorporation of features and embodiments mentioned above, in particular embodiments of the invention the functional group of the functionalised ceramic precursor is capable of chemically interacting with, for example electrostatically interacting with, the biomolecule. For example, the functionalised ceramic precursor may be an aminofunctional alkoxysilane. In certain embodiments, the aminofunctional ceramic precursor comprises an aminoalkylamino group. For example, the aminofunctional ceramic precursor may comprise 3-(2-aminoethylamino)propyl trimethoxysilane, 3-[2-(2-aminoethylamino)ethylamino]propyl trimethoxysilane, 3-(2-aminoethylamino)propyl triethoxysilane or 3-[2-(2-aminoethylamino)ethylamino]propyl triethoxysilane, or a mixture of any two or more of these.

The surfactant may again have an HLB of about 8 to about 16. It has been found that good results may be achieved with nonylphenol ethoxylate. The hydrophobic phase may additionally comprises a co-surfactant, such as an alcohol, for example 1-pentanol.

In the case of the preparation of nanoparticles the viscosity is not considered critical because micro-emulsions are formed (as opposed to normal emulsions), which are thermodynamically stable and consist of very small (≈10 nm) droplets.

In certain embodiments, the hydrophobic liquid comprises an alkane (e.g. from hexane (C6) to dodecane (C12)), a cycloalkane such as cyclohexane, aromatics (e.g. toluene, benzene) and blends such as kerosene.

The hydrophilic phase comprises water and a catalyst comprising an acid.

The biomolecule may be as described above. For example, it may be negatively charged or sufficiently large that it is incapable of passing through pores of the particles. The biomolecule may comprise an RNA, an antisense nucleotide, and antisense, an aptamer, a DNA, a protein, a glycoprotein, a polypeptide, a carbohydrate or a mixture or adduct of any two or more of these. In a particular embodiment the biomolecule comprises siRNA.

The process includes adjusting the pH of the emulsion, for example by addition of a base such as NaOH, KOH and NH₄OH prior to the addition of the biomolecule and the functionalised ceramic precursor to avoid denaturation of the biomolecule. Typically hydrolysis is conducted at low pH (such as 2) to ensure sufficient kinetics for the hydrolysis reaction while inhibiting condensation of the hydrolysed precursor. Before addition of the biomolecule, the pH is preferably increased to more neutral conditions (i.e. pH > 4).

Again, a polymer or complexing agent could be added such that it is disposed within the pores of the particles with the biomolecule to facilitate endosomal escape. Typically the polymer could be a polyethylinamine, a polylysine, or a polyhistidine or any substance that provides a proton sponge effect.

As with the previous aspect of the invention, the process may additionally comprise:
f) adding a surface treating agent to the emulsion following formation of the particles so as to surface treat the particles.

The surface treating agent may comprise a polyethylene glycol chain coupled to a binding group, said binding group being capable of binding the polyethylene glycol chain to the surface of the particles. For example, the surface treating agent may be a PEG-silane, such as trialkoxysilyl-PEG

The surface treating agent may comprise a targeting group for targeting a target in a patient. For example, the surface treating agent may comprise a trialkoxysilyl-PEG comprising the targeting group at the distal end of the PEG from the trialkoxysilane group.

The invention also provides for particles made by a process as described in any of the preceding paragraphs.

As noted above, the biomolecules may be indicated for prophylactic or therapeutic treatment of a disease, disorder or condition.

Accordingly, in an aspect of the invention there is provided a pharmaceutical composition comprising a particulate substance as disclosed herein together with a pharmaceutically acceptable carrier, diluent or excipient.

The pharmaceutically acceptable carrier, diluent or excipient may be a solid or liquid filler, solvent, diluent or encapsulating substance that may be safely used in systemic administration. Depending upon the particular route of administration, a variety of carriers well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates and pyrogen-free water. A useful reference describing pharmaceutically acceptable carriers, diluents and excipients is Remington's Pharmaceutical Sciences (Mack Publishing Co. N.J. USA, 1991).

Dosage forms include tablets, dispersions, suspensions, injections, solutions, syrups, troches, capsules, suppositories, aerosols, transdermal patches and the like. These dosage forms may also include injecting or implanting controlled releasing devices designed specifically for this purpose or other forms of implants modified to act additionally in this fashion.

Any safe route of administration may be employed for administering the particulate substance of the invention. For example, oral, rectal, parenteral, sublingual, buccal, intravenous, intra-articular, intra-muscular, intra-dermal, subcutaneous, inhalational, intraocular, intraperitoneal, intracerebroventricular, transdermal and the like may be employed.

In yet another aspect there is provided a particulate substance as disclosed herein, for use in treating a disease, disorder or condition in a mammal.

The disease, disorder or condition may be a genetic disease, disorder or condition (e.g. cystic fibrosis or Huntington's disease), a degenerative disease, disorder or condition (e.g. aged related macular degeneration), a cancer (e.g. solid tumors, sarcomas, lymphomas, myelomas, carcinomas, melanomas including cancers of the breast, cervix, lung and prostate, although without limitation thereto) a disease, disorder or condition of the immune system, inclusive of autoimmune diseases (e.g. Type 1 diabetes, multiple sclerosis, rheumatoid arthritis, systemic lupus erythematosus) and inflammatory conditions (e.g. asthma, inflammatory bowel disease, glomerulonephritis), a disease, condition or disorder caused by infection by a pathogen such as a virus (e.g. hepatitis C, influenza, respiratory syncytial virus infection, AIDS), a bacterium (e.g pneumonia, bacterial meningitis, whooping cough, tuberculosis, tetanus), protozoa (e.g. malaria) or a fungus (e.g Candida), a disease, disorder or condition of the circulatory system (e.g atherosclerosis, restenosis, hypercholesterolemia), a disease, disorder or condition of the endocrine system (e.g type II diabetes, osteoporosis, pancreatitis) or a neurological disease, disorder or condition (e.g. Alzheimer's disease, Parkinson's disease or epilepsy), although without limitation thereto.

The mammal may be a human or non-human mammal inclusive of performance animals (e.g. racehorses), domestic pets (e.g. dogs, cats) and livestock (e.g. cattle, horses, sheep, pigs), although without limitation thereto. Preferably, the mammal is a human.

### BRIEF DESCRIPTION OF THE DRAWINGS

Embodiments of the present invention will now be described, by way of an example only, with reference to the accompanying drawings. It should be appreciated that the following discussion should not be taken as limiting on the invention in any way. In the drawings:
Figure 1 is a flow chart of the preparation of the particles of the present invention;
Figure 2 shows TEMs of particles containing siRNA, made by the process of the invention;
Figure 3 shows particle size distributions of the particles;
Figure 4 shows further TEMs of the particles of the invention;
Figure 5 shows a graph illustrating the effect of particle charge and PEGylation on the release of fluorescent siDNA from the particles;
Figure 6 shows a graph illustrating release kinetics for different payloads in the particles;
Figure 7 shows an HPLC chromatogram of unencapsulated siRNA (red trace) and siRNA released from particles prepared according to this invention;
Figure 8 shows photographs of suspensions of the particles of the invention;
Figure 9 shows micrographs illustrating penetration of particles into the cells for particles having negative, neutral and positive charges;
Figure 10 shows micrographs illustrating retention of the cargo in particles having negative, neutral and positive charges;
Figures 11 and 12 shows micrographs illustrating that the cargo enters cells with the particles - Figure 11 shows the particles and Figure 12 shows the cargo;
Figure 13 shows the dispersal of siDNA in HEPG2 cells as a function of time;
Figure 14 shows the dispersal of siDNA in HeLa cells as a function of time;
Figure 15 shows the dispersal of siDNA in RAW264 cells as a function of time;
Figure 16 shows the dispersal of siDNA in cells as a function of time;
Figure 17 shows the effect of the particles of the present invention on activity of DPP4 in BJ fibroblasts;
Figure 18 shows detailed flowchart of a prototype method for encapsulation of oligonucleotides;
Figure 19 shows a flowchart of the preparation of the particles of the present invention, on the nano-scale;
Figure 20 shows FEG-SEM images of the particles made in accordance with the process illustrated in Figure 19;
Figure 21 shows phase and fluorescence images of particles labelled with fluoro-DNA; and
Figure 22 shows the penetration of nano particles in HeLa Cells.

### DETAILED DESCRIPTION OF THE INVENTION

Encapsulation and controlled release of siRNA from modified silica particles is described. The particles consist of amorphous silica (SiO₂) with a proportion of aminosilanes incorporated to aid cargo retention and cell penetration. The particles are surface modified for biocompatibility (circulating half-life ∼4h). The particles can penetrate mammalian cell membranes and release their cargo into the endosomal and intracellular spaces.

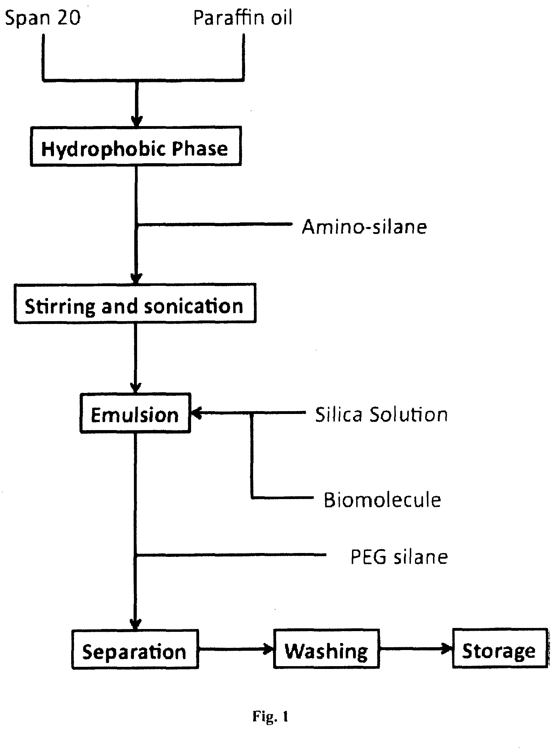

Fig. 1 illustrates the synthesis of the particles, including the encapsulation of siRNA (a representative biomolecule, which represents the cargo of the resulting particles). Thus with reference to Fig. 1, a hydrophobic continuous phase was made by combining 30 mL heavy paraffin oil and 4.5 g SPAN-20 (=500 mM). These were combined by stirring (30 minutes). Aminosilane (DATMS or TATMS, not APTES) was then added in sufficient quantity for the desired charge: for negative particles, no addition, for neutral particles, DATMS (1.5 uL = 1 mol% as silicon) and for positive particles, DATMS (15 uL = 10 mol% by silicon). The resulting mixture was then stirred for at least additional 10 minutes but no more than 60 minutes.

A silica solution was then prepared by combining 4 mL waterglass and 20 mL water. Sufficient cation exchange resin was added to the resulting mixture with stirring to bring the pH to 4.0. The silica solution was then decanted from the resin into a fresh container.

The hydrophobic phase (made as described above) was set up for simultaneous magnetic stirring and sonication (3/8" probe), and the stirrer activated. The sonicator was ramped to 70% power (-700 W) in preparation for combining the hydrophobic and hydrophilic phases.

5 mg cargo (250 uL 20mg/mL siRNA solution) was mixed with 1.25 mL of the silica solution prepared as described above. After 10 seconds sonication, the silica/cargo mixture was added into the sonicator active zone. Sonication was continued for 30 seconds and the sonicator was then deactivated. The emulsion was removed and introduced to a magnetic stirrer and was stirred for 1 hour. After this, PEG5000-silane (10 mg) was added to the mixture and the resulting particle suspension was stirred overnight.

Particles were collected from the emulsion by centrifugation (15 000xg for 10 minutes). The emulsion was then diluted with 0.5 volume cyclohexane to reduce its viscosity and washed twice with cyclohexane (about 40 mL) and twice with 100% ethanol (about 40 mL). Each wash step involved resuspending and collecting the particles and decanting the supernatant. The particles were finally resuspended in 5 mL of 100% ethanol for storage at -20°C or 4°C. The particles may be stored for several months at 4°C without substantial loss of biological activity, however lower temperature storage will provide even longer term storage.

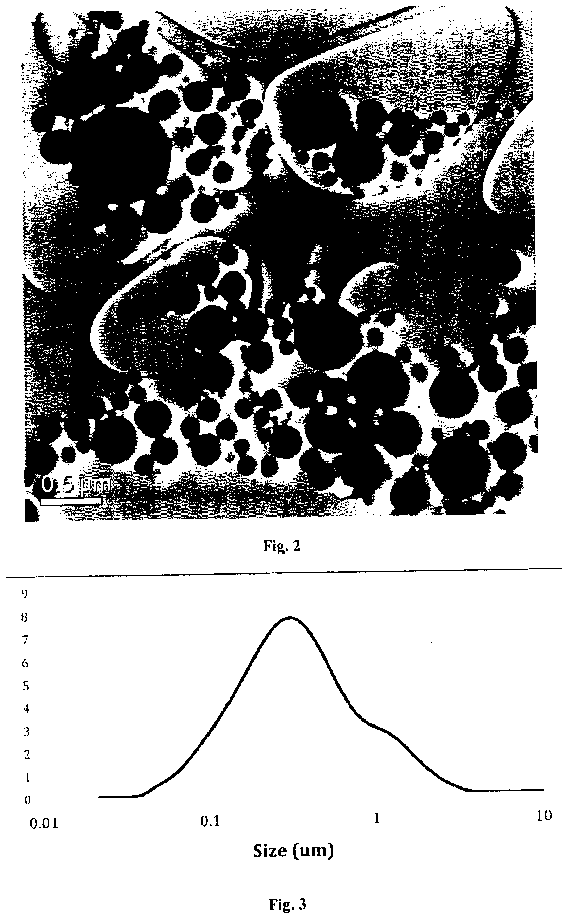

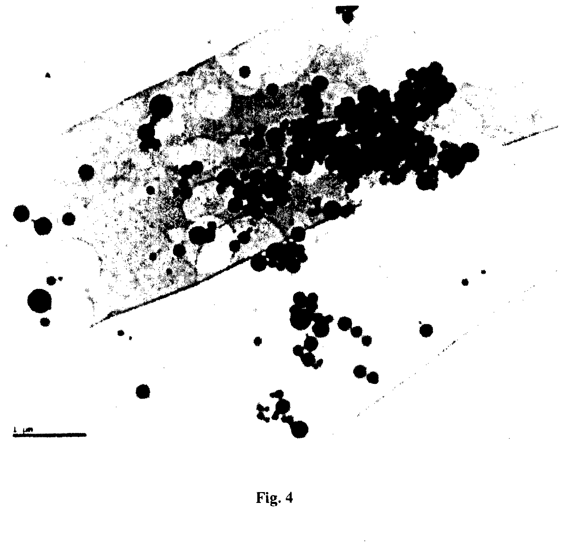

The above method provides particles ranging in particle size from 100-1000 nm, with a mass-weighted mean diameter (d_{0.5}) of about 300 nm. These are shown in Figs. 2 and 4. Figure 3 shows particle size distributions of the particles. The shoulder at about 1 micron probably represents a minor amount of aggregated particles. The above method has been used in the studies described below, however modifications of the method have produced dispersed particles with d_{0.5}<150 nm.

Particles were prepared with different charges by varying the amount and/or type of aminosilane added. DATMS (aminoethylaminopropyltrimethoxysilane: 2 nitrogen atoms per molecule) was used as the standard. APTES (aminopropyltrimethoxysilane: 1 nitrogen atom per molecule) was much less effective and TATMS (aminoethylaminoethylaminopropyltrimethoxysilane: three nitrogen atoms per molecule) showed similar results to DATMS.

As noted above, the aminosilane was added to the hydrophobic phase and then transferred to the hydrophilic phase by hydrophilic transfer. Due to partitioning between the phases the amount of aminosilane incorporated was less than the amount added. It was found that direct addition of the aminosilane to the hydrophilic phase (i.e. combination with the waterglass) was not practicable at acidic pH as this caused premature gelation.

The charge of the particles was measured at pH 7.0 in 10 mM MOPS (3-N- morpholinopropane sulfonic acid buffer). Zeta potentials for the particles were as follows:
Native (no aminosilane): ζ ≤ -30 mV
Neutral (1% DATMS): -5 mv < ζ < 5 mV
Positive (10 % DATMS): ζ ≥ +10 mV
Despite the use of an indirect measurement method, the measured charge was quite repeatable between batches.

The percentage encapsulation efficiency (EE) was determined by comparison of the theoretical loading of the siRNA (determined from the amount added) with the actual loading as measured by the amount released. Results are shown below:

### Theoretical loading: 5%

EE (from 1 mg/mL release)
   Batch 1: 85% +/- 5%
EE (from 0.1 mg/mL release)
   Batch 1: 80% +/- 2%
   Batch 2: 85% +/- 10%
Actual loading 4.2%

### Theoretical loading: 10%

EE 75%, loading 7.5%

Thus the higher the quantity of RNA introduced the lower the encapsulation efficiency.

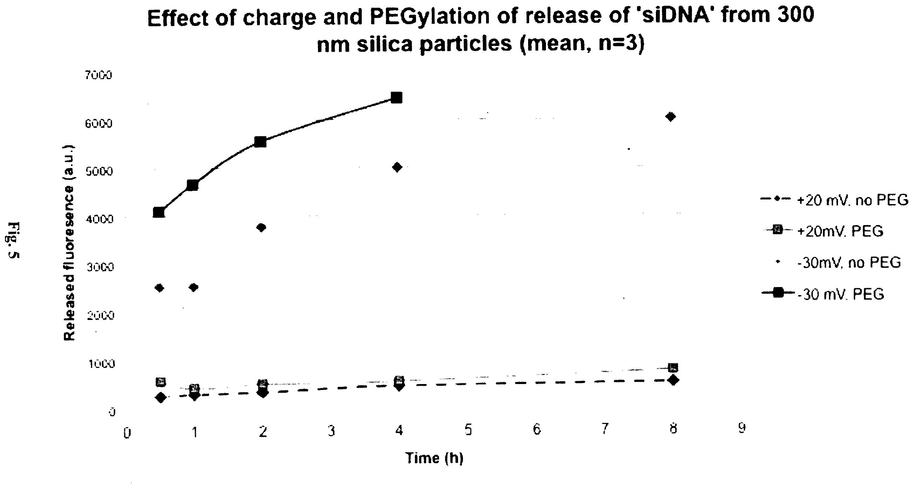

Fig. 5 shows the effect of the release of a fluorescent labelled siDNA from silica particles of different charge and surface modification. As discussed above, particle charge may be manipulated by changing the amount of aminosilane used. Release from positively charged particles was very much slower than from negatively charged particles, as predicted by the expected attraction between positively charged particles and negatively charged payload. For the negatively charged particles, the presence of PEG on the surface of the particles appears to accelerate the release of the payload.

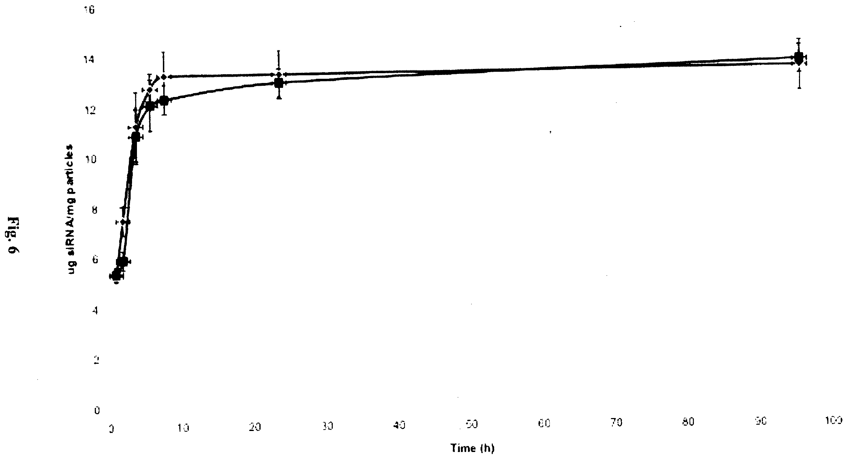

Release of the payload from the particles is thought to be primarily by dissolution of the particle matrix. At high concentrations in aqueous media (≥ about 1mg/mL particles), leaching of cargo from the particles is limited to that mediated by particle dissolution, i.e. the solution can reach saturation in the particle matrix, thereby limiting the release of the payload. This is shown in Fig. 6, in which relatively rapid release of both active (dot point values) and scrambled (square point values) siRNA molecules occurs up to a limit dictated by the solubility of the silica matrix. It should be noted that this is not evident in Fig. 5 as the concentrations of particles was different. At concentrations substantially below the solubility limit of silica (approximately 100µg/mL), or in situations in which the release liquid is continuously refreshed, full dissolution of particles occurs over about 12-24 hours. Particles made as described above were stored for 36 days at 253K in 96% ethanol. After storage, the particles were completely dissolved in RNase-free water. Elution of the resulting liquid on HPLC showed a very similar profile to that of unencapsulated siRNA in a buffer solution, indicating that encapsulation and release did not significantly affect the siRNA.

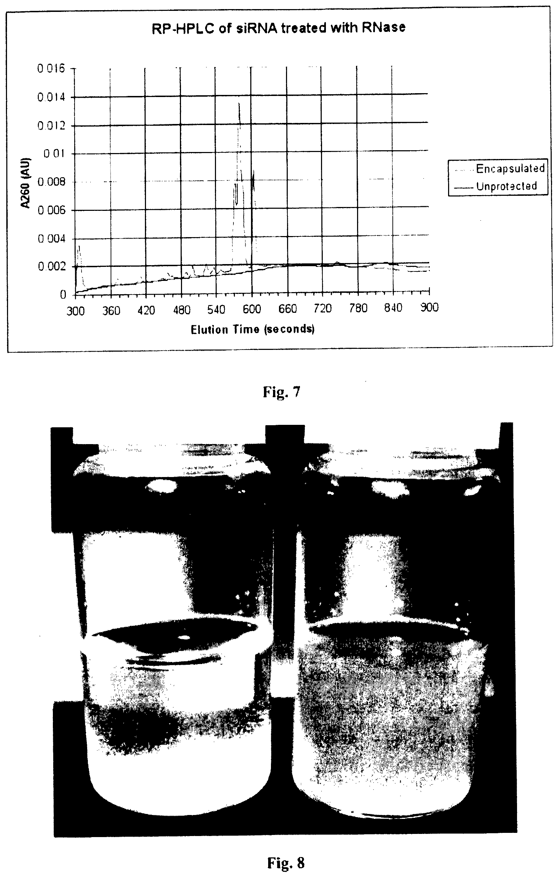

Figure 7 shows an HPLC chromatogram of un-encapsulated siRNA and siRNA released from particles prepared according to this invention. Both particles and reference (unencapsulated siRNA) were treated with RNase A for 15 minutes then washed three times with PBS before suspension in PBS containing an RNase inhibitor. The material released from silica particles shows intact RNA. Similar digestion of unencapsulated siRNA resulted in complete destruction. These experiments demonstrate the capacity of the particles to protect the encapsulated biomolecule against enzymatic degradation.

Fig. 8 shows photographs of the particles suspended at 3 mg/L against either PBS (left) or against 50% murine serum in PBS (right). After overnight incubation no visible aggregation occurred. Particles were also suspended at 1, 3, 10 mg/kg in 1500 ppm BSA and then incubated for 2 hours. Particle size was then determined by Mastersizer (Mie scattering), revealing no time- or concentration-mediated shift in size profile.

In conclusion, cargo loadings of 4% are routinely achievable, and loading of about 8% has been demonstrated. Encapsulation efficiency of >80% is routinely achievable. Retention of the biomolecule in the particles appears to be mediated primarily by electrostatic forces, yielding dissolution-limited release characteristics at physiological pH (i.e. the release take place predominantly by dissolution of the matrix). Cargo retention characteristics have been shown to be unchanged after 40 days storage at -20°C in 96% EtOH.

### Uptake into Mammalian Cells

The influence of particle charge on cell penetration and cargo retention, and the time course of uptake into cells and endosomal escape were investigated.

Particles covalently labelled with RITC (rhodamine isothiocyanate) and carrying a DNA with an siRNA-type sequence and labelled with FITC (fluorescene isothiocyanate) were synthesised. Cells (NIH3T3, HeLa, HEPG2) were cultured to 50% confluence and particles as described above (about 30µg/ml, equivalent to 100nM DNA) were added directly to the culture medium. After 40h, the cultures were washed once with PBS (phosphate buffered saline) in order to remove particles which had not penetrated into cells and then imaged by epifluorescent microscopy.

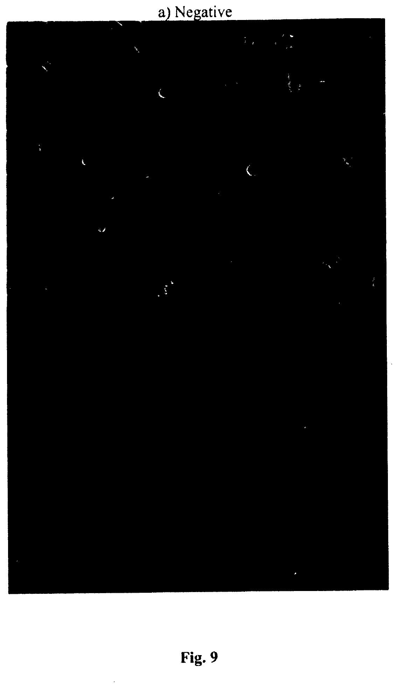

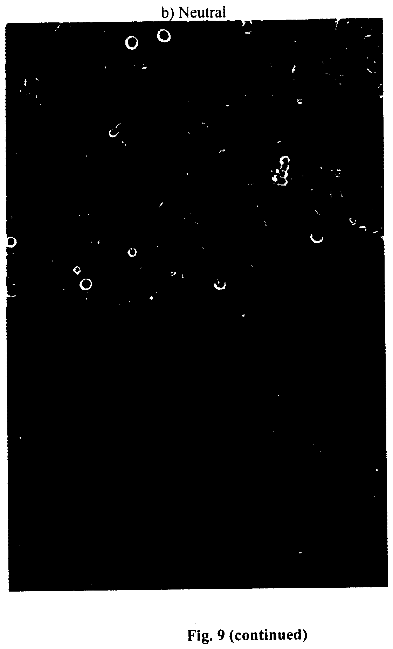

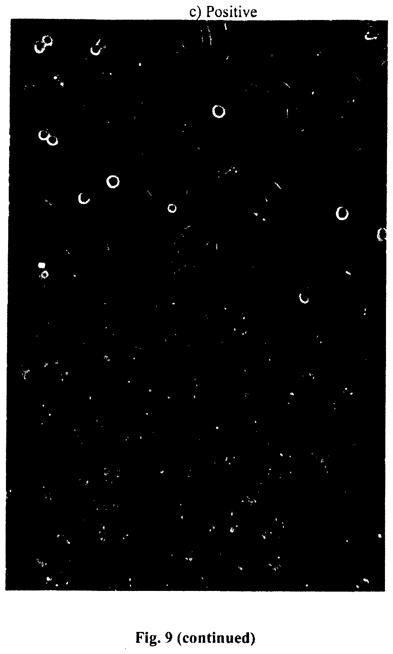

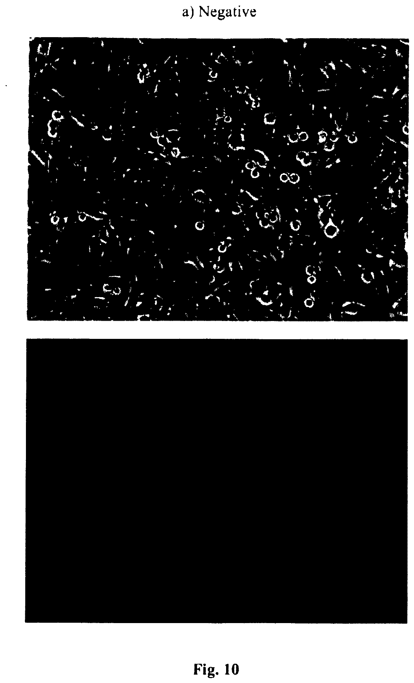

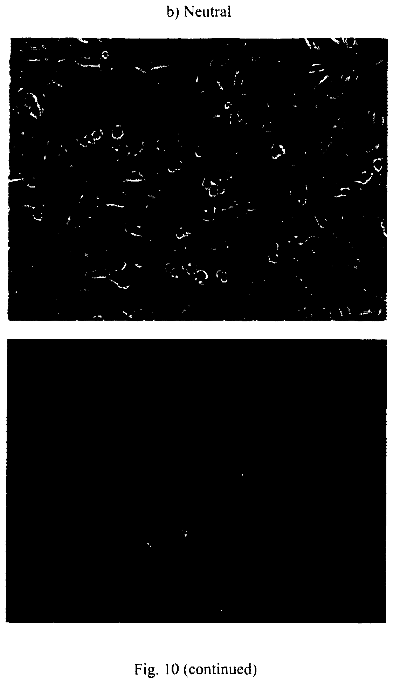

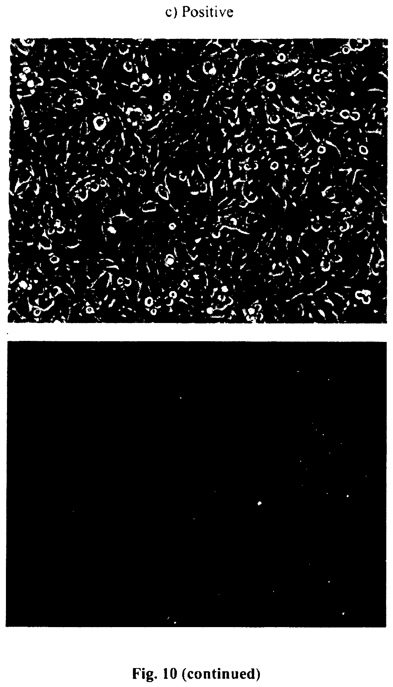

Fig. 9 shows the results of monitoring the RITC label: in each pair of images, the top image is a phase contrast image and the bottom image is a fluorescence image. Fig. 9 indicates that with increasing positive charge on the particles, the more the particles are taken up by the cells. Thus a positive charge on the particles assists not only in binding the payload but also assists with particle uptake into cells. Fig. 10 illustrates that the cargo is more effectively retained in positively charged particles as they are taken up by cells compared to neutral or negatively charged particles. This figure shows siDNA retention by charge. In each pair of images, the top image is a phase contrast image and the bottom image is an siDNA fluorescence image.

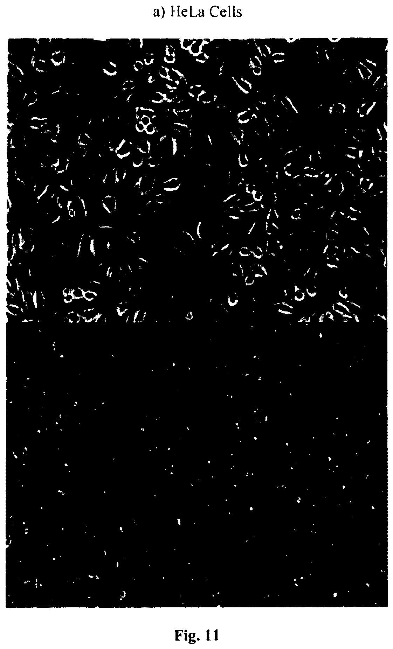

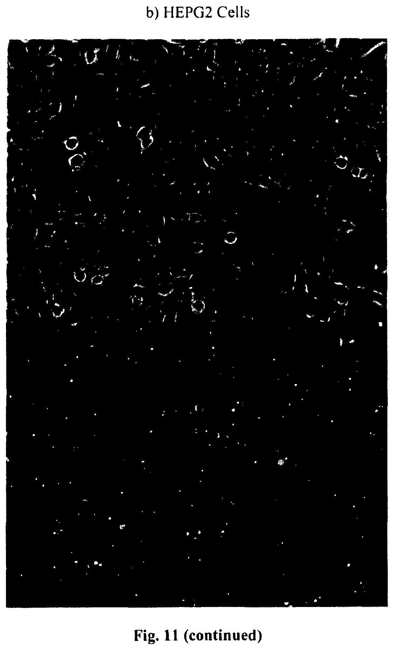

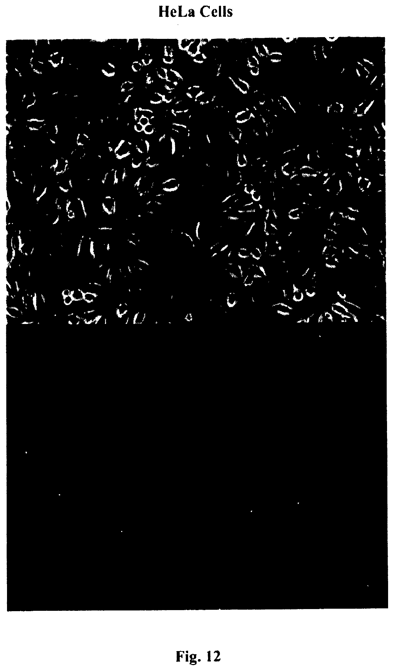

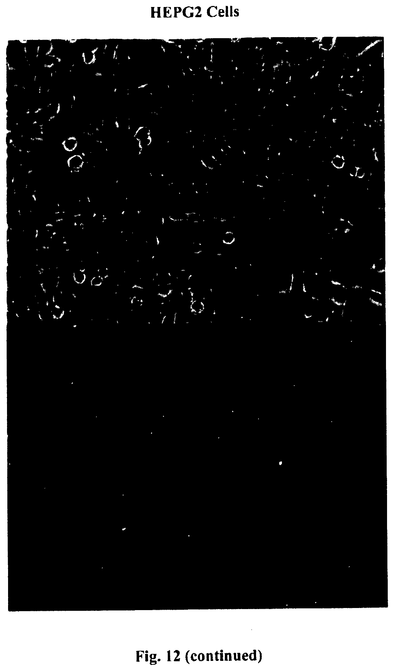

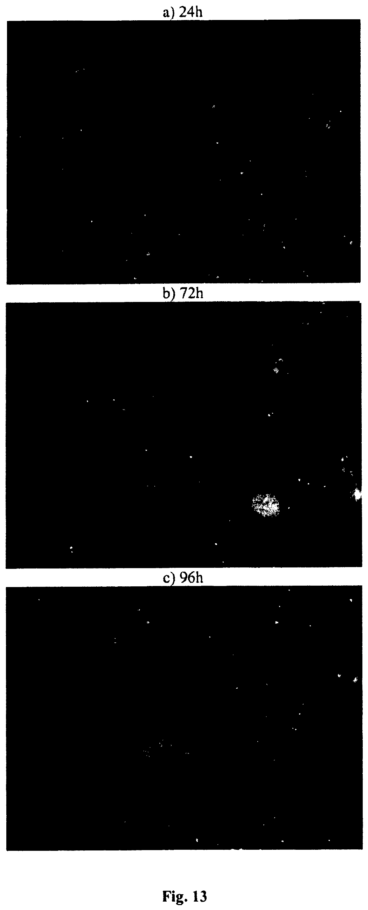

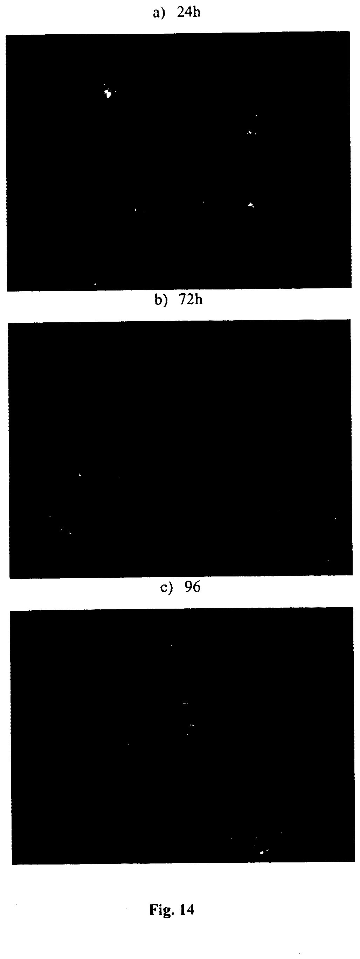

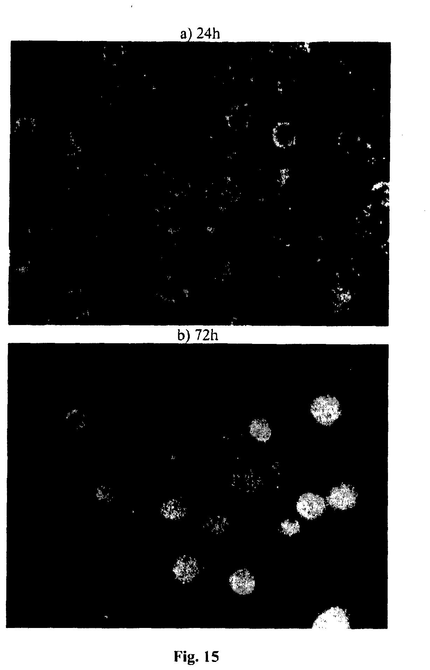

Fig. 11 shows the uptake of particles into two different cell lines (i.e. the particle distribution), and Fig. 12 shows micrographs of the same samples but with the labelled payload highlighted (i.e. the cargo distribution). In each pair of images in both of these figures, the top image is a phase contrast image. In Fig. 11 in each pair the bottom image is an RITC fluorescence (red channel) image, and in Fig. 12 in each pair the bottom image is a fluorescence image of siDNA (green channel). By comparison of these two figures it can be determined that the siRNA is retained in the particles as the particles penetrate into the cells. Collocation of the green and red dots inside the cell means that the silica has penetrated inside (red channel dots=silica particles) while retaining its fluorescent DNA cargo (green channel dots =DNA), thus demonstrating that the DNA has been successfully transfected inside the cells. Figures 13 to 15 show the time course of introduction of labelled siDNA into various cell lines (Fig. 13: HEPG2; Fig. 14: HeLa; Fig. 15: RAW264) by way of the particles of the invention. Each figure shows the fluorescence distribution at each time posttreatment in the cells. In each case, it can be seen that at shorter time periods the siDNA is located primarily in small regions, representing the localisation within particles located in the cells. At longer time periods the siDNA spreads into larger regions, representing the release from the particles by dissolution of the particle matrix and distribution through the cells.

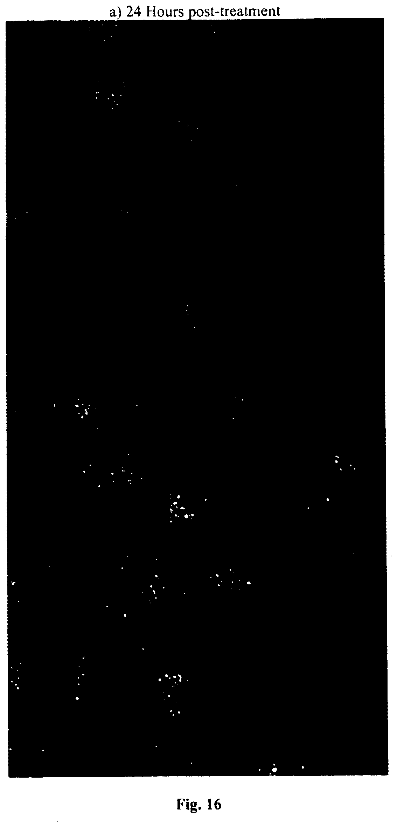

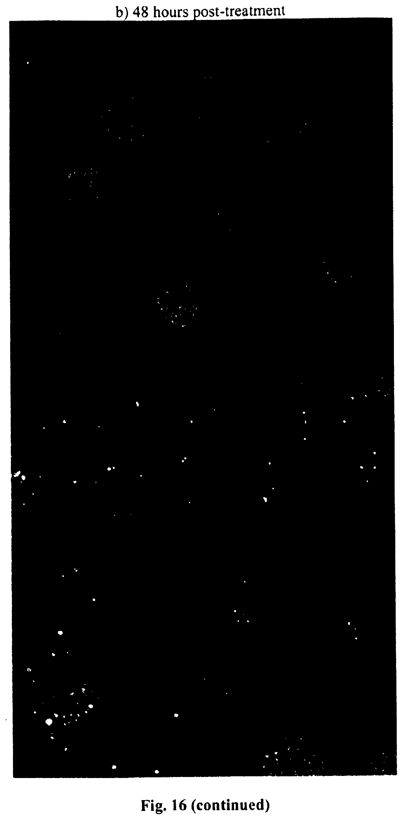

Figure 16 shows a similar experiment using confocal microscopy. In Fig. 16, the top image of each pair shows the nucleus stain (blue channel) and the bottom image shows the siDNA fluorescence (green channel). Thus HeLa cells were plated onto poly-lysine-coated coverslips at 25% confluence. These were treated for 24 or 48h with RITC-modified particles carrying FAM-DNA. They were then washed with PBS and fixed with 3.7% formaldehyde in PBS. They were then stained with 1.2 µg/mL Hoescht 33342 in isotonic saline, mounted on slides with Gelmount and acrylic and imaged with confocal microscope at 100x magnification. The images are 150 x 150 µm, z-axis slice depth 350 nm. The well defined approximately round structures represent nuclear DNA. After 24 hours there are a large number of small bright regions, representing the payload localized within the particles. A small amount of diffuse lighting represents a small amount of released payload. After 48 hours the point sources have largely disappeared, representing the dissolution of the particles. Instead, each cell has a diffuse halo of light region representing the released payload within the cell.

### Gene Knockdown Studies

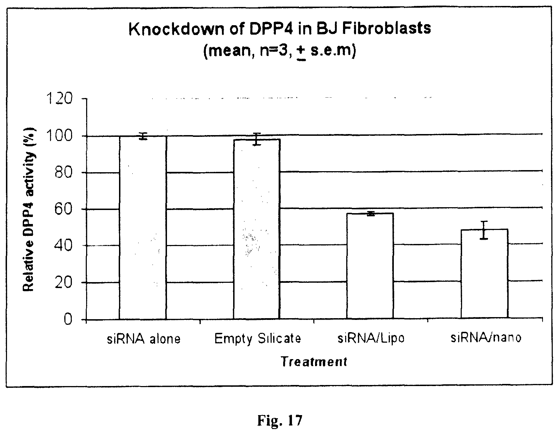

Fig. 17 shows the results of an experiment to show the effectiveness of the present loaded particles in knockdown (i.e. inhibition of gene expression). This experiment looked at effectiveness knockdown of DPP4 in human BJ fibroblasts. siRNA alone was ineffective, possibly due to inactivation by RNase present in the system. Unsurprisingly, unloaded silica particles were also ineffective. The measurement labeled siRNA/Lipo refers to siRNA transfected by means of Lipofectamine®, which is known to transfect oligonucleotides across the cell membrane. This system has the disadvantages that it is toxic and does not provide protection for the siRNA from enzymatic attack. The measurement labeled siRNA/nano represents siRNA encapsulated in particles according to the present invention. In each case in which siRNA was present, it was used at about 200nM. The results show that the encapsulated siRNA was effective at knockdown at this concentration, and was in fact slightly more effective than siRNA with Lipofectamine.

### In Vitro conclusions

- Encapsulation of biomolecules into particles according to the present invention can protect the biomolecules from enzymatic degradation.
- when applied to cells under normal culture conditions, the particles loaded with biomolecules are capable of penetrating the cytoplasmic membrane and delivering their cargo to the intracellular space.
- delivery of biomolecules via the particles of the invention to tissue culture cells results in dose-dependant reduction of mRNA levels in those cells.
- doses of siRNA encapsulated in particles sufficient to result in >50% reduction in mRNA levels show no significant toxicity *in vitro.*

### Additional Results - Synthesis of particles

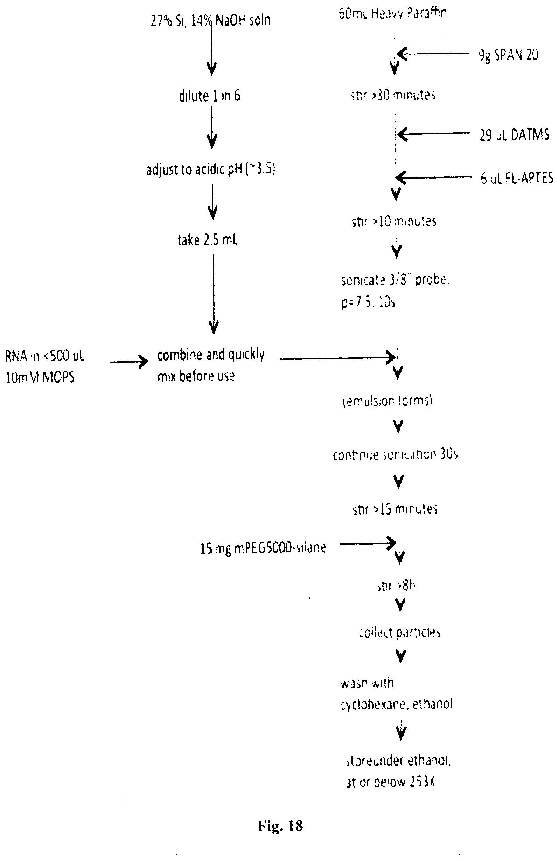

The general synthetic method is described by the flow diagram in Figure 18. Particle formation was extremely rapid on addition of the aqueous precursor to the surfactant solution. However, in general at least 12 hours was allowed between formation of the emulsion and particle collection.

Retention of oligonucleotides is strongly influenced by electrostatic interactions between the cargo and the aminosilane component of the particles. This makes the quantity and type of substitution, and also the pH of both formation and release critical factors in determining encapsulation, retention and release characteristics.

### Parameters

The surfactant used in this example was Sorbitan monolaurate (Span® 20). The surfactant concentration used was about 17% by mass. The hydrophobic phase was heavy liquid paraffin, this giving the smallest particles of those tested. Particle size was reduced to a value acceptable for intravenous injection by a combination of magnetic stirring and sonication.

The preferred aminosilane used to enhance cargo retention was DATMS (aminoethylaminopropyl trimethoxysilane). Experiments with APTES (aminopropyl triethoxysilane) and TATMS (aminoethylaminoethylaminopropyl trimethoxysilane) show they also have this effect to a lesser or greater extent, and may be of use in fine-tuning retention/release characteristics. Cargo loading is expected to affect particle zeta potential, and aminosilane modification is expected to affect maximum loading.

The pH of minimum stability for waterglass is approximately 5.5, which represents the pH of maximum stability for RNA. If the silicate solution is too close to neutral, the precursor will spontaneously gel before it can be used for particle synthesis. If the solution is too acidic, significant degradation of the nucleotide cargo will occur. With RNA cargos a precursor pH of 3.75-4.00 has proved to be suitable if somewhat difficult to handle. DNA, LNA, or other modified oligonucleotides may allow for more acidic (and hence more stable) precursor solutions.

### Example - Figure 18

### Encapsulation of siRNA into particles modified with DATMS, rhodamine, and mPEG-5000:

15g of Dowex 50W was stirred with 100 mL 5M HCI for 30 minutes to convert the resin to the active, protonated form. The resin was then recovered by vacuum-assisted filtration into a sintered-glass filter funnel, wherein it was washed twice with 100 mL milliQ water to remove residual HCI.

9 grams Span® 20 was weighed into a Teflon beaker and 60 mL liquid paraffin added. The resulting mixture was stirred for about 30 minutes to complete dissolution of the Span® 20 in the paraffin. 29 µL DATMS liquid and 6 µL 10% Rhodamine-APTES in 2-propanone was added to the stirred surfactant solution.

4.0 mL sodium silicate solution was added to 28 mL milliQ water. 8.0 mL of this solution was set aside for subsequent titration of main volume.

Using a pH probe to continually monitor the solution pH, activated cationic exchange resin was added to reduce the pH of the silicate mixture to approximately 3.5. The silicate solution was decanted from the resin and the pH rechecked.

2.5 mL of this precursor solution was transferred to a 5 mL plastic tube . An appropriate volume (<0.5 mL) of cargo RNA solution was transferred to a 1.5 mL tube.

The cargo RNA solution was pipetted into the silicate precursor. The RNA/silicate mixture was pipetted into the surfactant solution and sonication was continued with stirring for 25 seconds.

The resultant emulsion was rapidly stirred for 15 minutes. 15 mg mPEG-5000 silane powder was then added to the emulsion and the resulting mixture stirred overnight.

The mixture was then centrifuged for 5 minutes at >2000 x g to isolate the particles. The particles were then washed twice with cyclohexane to remove paraffin and surfactant, centrifuging after each wash, and then washed once more with ethanol. The particles were collected by centrifugation, supernatant decanted, and the particles resuspended in 10 mL ethanol.

The typical weight of product obtained was 200 mg. The typical encapsulation efficiency was >80%. The typical zeta of particles at pH 7.4 was +20 mV. The typical reduction of protein binding to particles when compared to native silicate particles (a measure of PEGylation density) was >90%.

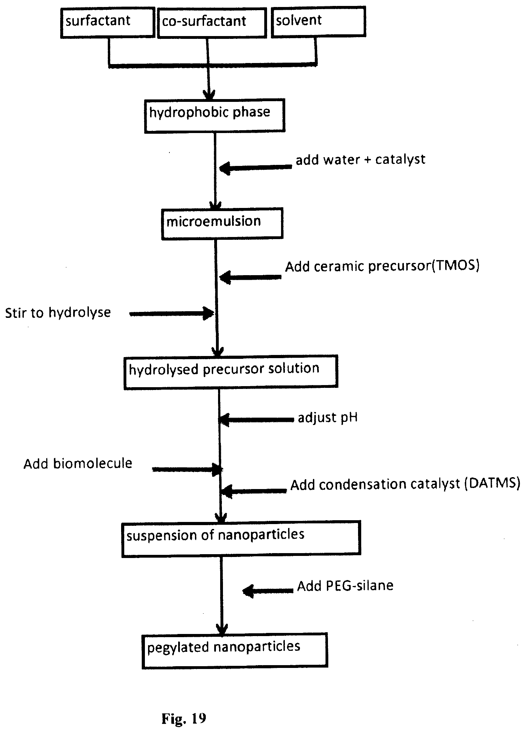

### Examples - Figure 19

### A. Microemulsion synthesis of particles for biomolecule encapsulation

0.381 g of NP9 was dissolved in 3 mL of cyclohexane (0.2 mol/L) by stirring (magnetic) in a glass vial and 0.065 mL of 1-pentanol subsequently added as a co-surfactant with continued stirring (0.2 mol/L). The resultant solution constituted the hydrophobic phase.

0.013 mL of 0.01M HNO₃ was added to act as an acid catalyst, constituting the hydrophilic phase, and the solution stirred for 20 minutes to homogenise. This resulted in formation of a microemulsion.

0.018 mL of tetramethylorthosilicate (TMOS) was added and the resulting solution stirred for 66 hours to hydrolyse the TMOS and provide a hydrolysed precursor solution.

0.013 mL of 0.01M NaOH was added and stirred for 5 minutes to adjust the pH to greater than about 4.

Addition of a biomolecule was simulated by addition of 0.010 mL of water with stirring. As a functionalised ceramic precursor, 0.003 mL of 3-(2-aminoethylamino)propyltrimethoxysilane was added and the mixture stirred for 6.5 hours, over which time the solution became progressively more cloudy. This provided a suspension of nanoparticles.

5 mg of mPEG-silane (MW = 5000) was added and the solution left stirring for 15 hours. The solution was then centrifuged (13,000 for 1 minute) to isolate the particles, which were then washed three times with 2 mL of ethanol, and suspended in 2 mL ethanol.

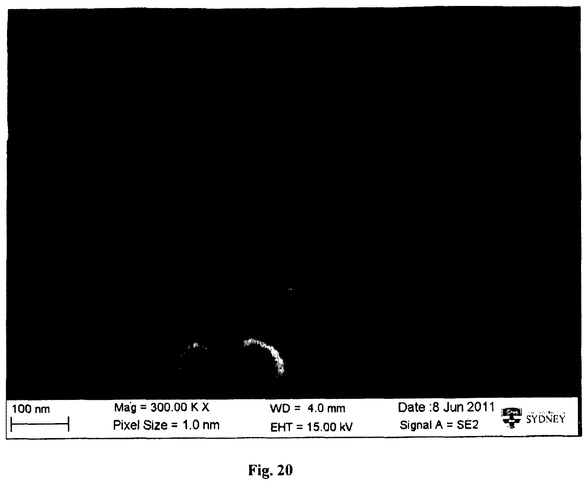

The particles were imaged by FEG-SEM, which showed a size range of 30 - 100 nm. Reference is made to Figure 20.

### B. Microemulsion synthesis of particles for biomolecule encapsulation

0.636 g of NP9 was dissolved in 5 mL of cyclohexane (0.2 mol/L) by stirring (magnetic) in a glass vial. 0.109 mL of 1-pentanol was added as a co-surfactant with continued stirring (0.2 mol/L). 1.14 mL of the cyclohexane/NP9/1-pentanol solution was pipetted into a second glass vial (x2).

0.011 mL of 0.01M HNO₃ was added to the subsamples above and the solutions stirred for 40 minutes to homogenise, forming a microemulsion.

0.0125 mL (0.08 mMol) of tetramethylorthosilicate was added to the subsamples and the resulting solutions stirred for 17.5 hours to hydrolyse the TMOS. 0.011 mL of 0.01M NaOH was added to both samples and they were then stirred for 5 minutes to adjust the pH to greater than about 4.

0.006 mL of fluoro-DNA solution (FITC-labelled DPP4 (21 base pair)), 0.5 mg/mL in water) was added with stirring to one sample, and 0.006 mL of water was added to the second sample with stirring.

0.002 mL (0.009 mMol) of 3-(2-aminoethylamino)propyltrimethoxysilane was added as the functionalised ceramic precursor to each sample and the mixtures stirred for 6 hours.

0.8 mg of mPEG-silane (MW = 5000) was added to each sample, and the samples were then left stirring for 18 hours. 1 mL of acetone was added to each sample and the solutions stirred for 10 minutes.

The solutions were then centrifuged (13,000 for 1 minute) to isolate the particles, which were then washed three times with 2 mL of ethanol. The sample containing fluoro-DNA (CS11-0028) was suspended in 2 mL of ethanol. The sample made using water only (CS11-0029) was dried at 40 °C and weighed as 7.3 mg.

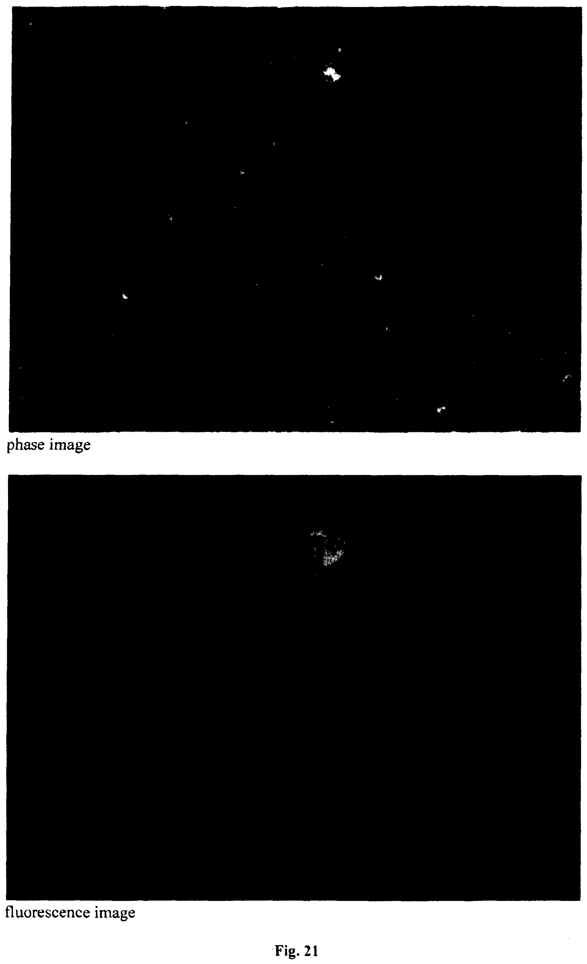

Several drops of the particles labelled with fluoro-DNA were dried on a microscope slide and imaged using a fluorescence microscope equipped with a FITC filter at 40 x magnification and 4 second exposure. Reference is made to Figure 21.

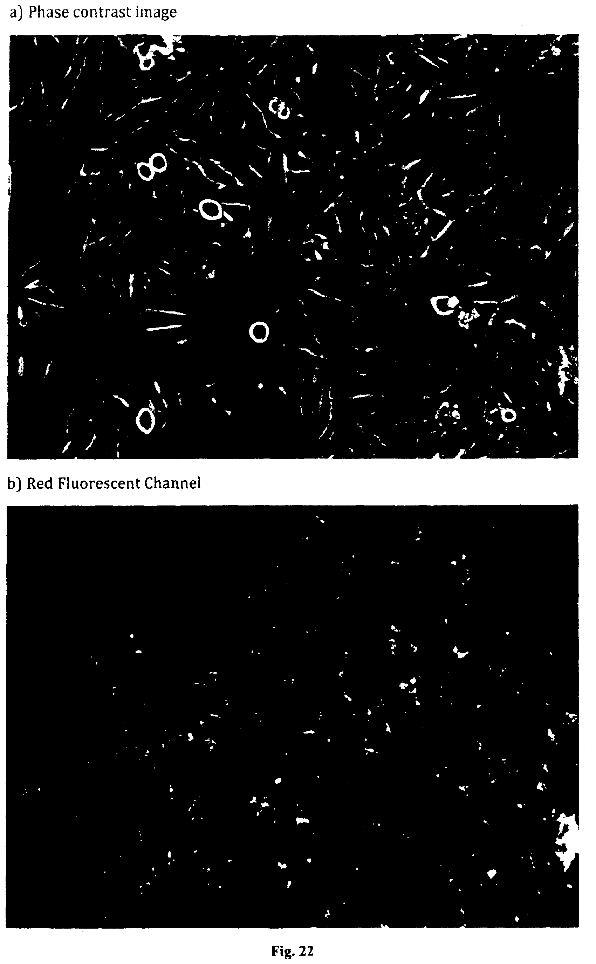

Figure 22 illustrates the transfection of cultured human hepatocytes with AlexaFluor-633 labelled silica nanoparticles. Cells were treated for 24 hours before imaging.

### Further Example

Particles covalently labelled with FITC (fluorosceine isothiocyanate) and carrying a Phycoerythrin payload will be synthesised. HeLa cells will be cultured to 50% confluence and particles as described above (about 30µg/ml) will be added directly to the culture medium. After 40h, the cultures will be washed once with PBS (phosphate buffered saline) in order to remove particles which had not penetrated into cells and then imaged by epifluorescent microscopy to thereby monitor intracellular release of the delivered Phycoerythrin.

Unless the context requires otherwise or specifically stated to the contrary, integers, steps or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of steps, elements or integers. Thus, in the context of this specification, the term "comprising" is used in an inclusive sense and thus should be understood as meaning "including principally, but not necessarily solely".

## Claims

1. A particulate substance comprising:
particles of a ceramic matrix bearing an aminofunctional group, the aminofunctional group being capable of promoting penetration of the particles into cells and being distributed homogeneously throughout said particles; and
a biomolecule disposed within pores of the particles, the biomolecule being releasable from the particles by dissolution of the ceramic matrix.

2. The particulate substance of claim 1, wherein the functional group chemically interacts with the biomolecule to substantially prevent leaching.

3. The particulate substance of claim 1 or 2, wherein polyethylene glycol chains are coupled to the surface of the particles.

4. The particulate substance of any one of claims 1 to 3, wherein a targeting group is coupled to the surface of the particles.

5. The particulate substance of any one of claims 1 to 4, wherein said particles have a mean particle size of 20 to 100nm, and a pore size of from 1 to 50nm.

6. The particulate substance of any one of claims 1 to 5, wherein a polymer or complexing agent is disposed in the pores of the particles with the biomolecule and provides a proton sponge effect.

7. A process for making particles comprising a biomolecule disposed in pores thereof, said process comprising:
a) combining:
a hydrophobic phase comprising a hydrophobic liquid, a first ceramic precursor and a surfactant; and
a hydrophilic phase comprising a hydrophilic liquid, a second ceramic precursor and the biomolecule,
so as to form an emulsion comprising droplets of the hydrophilic phase dispersed in the hydrophobic phase; and
b) agitating the emulsion as the particles form inside the droplets; wherein the first ceramic precursor comprises an aminofunctional group which is capable of promoting penetration of the particles into cells.

8. A process for making particles comprising a biomolecule disposed in pores thereof, said process comprising:
a) combining:
a hydrophobic phase comprising a hydrophobic liquid and a surfactant; and
a hydrophilic phase comprising a hydrophilic liquid comprising water and a catalyst comprising an acid,
so as to form an emulsion comprising droplets of the hydrophilic phase dispersed in the hydrophobic phase;
b) adding a ceramic precursor to the emulsion and hydrolysing the ceramic precursor;
c) adjusting the pH of the hydrophilic phase to a range suitable for the biomolecule;
d) adding the biomolecule and a functionalised ceramic precursor to the emulsion; and
e) agitating the emulsion as the particles form inside the droplets, wherein the functionalised ceramic precursor comprises an aminofunctional group which is capable of promoting penetration of the particles into cells.

9. The process of claim 7 or 8, wherein the aminofunctional group of the first ceramic precursor or functionalised ceramic precursor is capable of chemically interacting with, for example electrostatically interacting with, the biomolecule.

10. The process of claim any one of claims 7 to 9, wherein the first ceramic precursor or functionalised ceramic precursor is an aminofunctional alkoxysilane.

11. The process of claim 7, wherein the first ceramic precursor is a base and the hydrophilic phase has a pH below the pKₐ of the first ceramic precursor, the process comprising the steps of:
combining the hydrophilic liquid and the second ceramic precursor;
adjusting the pH to below the pKₐ of the first ceramic precursor; and
adding the biomolecule,
so as to form the hydrophilic phase, said steps being conducted prior to step a).

12. The process of claim 11, wherein the step of adjusting the pH comprises exposing a solution of the second ceramic precursor in the hydrophilic liquid to a cation exchange resin and then separating the solution from the resin once the pH of the solution has reached a desired pH below the pKₐ of the first ceramic precursor.

13. The process of any one of claims 7 to 12, wherein the biomolecule is negatively charged or is sufficiently large that it is incapable of passing through pores of the particles.

14. The process of any one of claims 7 to 13 additionally comprising:
adding a surface treating agent to the emulsion following formation of the particles so as to surface treat the particles.

15. The process of claim 14, wherein the surface treating agent is a PEG-silane, such as a trialkoxysilyl-PEG.

16. The process of claim 14 or 15, wherein the surface treating agent comprises a targeting group for targeting a target in a patient.

17. The process of any one of claims 7 to 16, wherein a polymer or complexing agent is added such that it is disposed within the pores of the particles with the biomolecule, and wherein the polymer is a polyethylinamine, a polylysine, or a polyhistidine or a substance that provides a proton sponge effect.

18. The process of claim 8, including adjusting the pH of the emulsion to greater than 4, for example by addition of a base, such as NaOH, KOH and NH₄OH, prior to the addition of the biomolecule and the functionalised ceramic precursor.

19. A pharmaceutical composition comprising a particulate substance of any one of claims 1 to 6, together with a pharmaceutically acceptable carrier, diluent or excipient.

20. A particulate substance of any one of claims 1 to 6, for use in treating a disease, disorder or condition in a mammal.

## Patentansprüche

1. Partikuläre Substanz, umfassend:
Partikel einer Keramikmatrix, die eine aminofunktionale Gruppe tragen, wobei die aminofunktionale Gruppe in der Lage ist, das Eindringen der Partikel in Zellen zu fördern, und homogen über die Partikel hinweg verteilt ist; und
ein Biomolekül, das innerhalb von Poren der Partikel angeordnet ist, wobei das Biomolekül durch Auflösung der Keramikmatrix aus den Partikeln freigesetzt werden kann.

2. Partikuläre Substanz nach Anspruch 1, wobei die funktionale Gruppe chemisch mit dem Biomolekül interagiert, um Auslaugung im Wesentlichen zu verhindern.

3. Partikuläre Substanz nach Anspruch 1 oder 2, wobei Polyethylenglycol-Ketten mit der Oberfläche der Partikel gekoppelt sind.

4. Partikuläre Substanz nach einem der Ansprüche 1 bis 3, wobei eine Zielgruppe mit der Oberfläche der Partikel gekoppelt ist.

5. Partikuläre Substanz nach einem der Ansprüche 1 bis 4, wobei die Partikel eine mittlere Partikelgröße von 20 bis 100 nm und eine Porengröße von 1 bis 50 nm aufweisen.

6. Partikuläre Substanz nach einem der Ansprüche 1 bis 5, wobei ein Polymer oder ein Komplexbildner in den Poren der Partikel mit dem Biomolekül angeordnet ist und einen Protonenschwammeffekt bereitstellt.

7. Verfahren zum Herstellen von Partikeln, umfassend ein Biomolekül, das in Poren davon angeordnet ist, wobei das Verfahren Folgendes umfasst:
a) Kombinieren:
einer hydrophoben Phase, umfassend eine hydrophobe Flüssigkeit, einen ersten Keramikvorläufer und ein Benetzungsmittel; und
einer hydrophilen Phase, umfassend eine hydrophile Flüssigkeit, einen zweiten Keramikvorläufer und das Biomolekül,
um so eine Emulsion zu bilden, umfassend Tröpfchen der hydrophilen Phase, dispergiert in der hydrophoben Phase; und
b) Rühren der Emulsion, wenn sich die Partikel innerhalb der Tröpfchen bilden;
wobei der erste Keramikvorläufer eine aminofunktionale Gruppe umfasst, die in der Lage ist, das Eindringen der Partikel in Zellen zu fördern.

8. Verfahren zum Herstellen von Partikeln, umfassend ein Biomolekül, das in Poren davon angeordnet ist, wobei das Verfahren Folgendes umfasst:
a) Kombinieren:
einer hydrophoben Phase, umfassend eine hydrophobe Flüssigkeit und ein Benetzungsmittel; und
einer hydrophilen Phase, umfassend eine hydrophile Flüssigkeit, umfassend Wasser und einen Katalysator, umfassend eine Säure,
um so eine Emulsion zu bilden, umfassend Tröpfchen der hydrophilen Phase, dispergiert in der hydrophoben Phase;
b) Hinzufügen eines Keramikvorläufers zu der Emulsion und Hydrolysieren des Keramikvorläufers;
c) Einstellen des pH-Werts der hydrophilen Phase auf einen Bereich, der für das Biomolekül geeignet ist;
d) Hinzufügen des Biomoleküls und eines funktionalisierten Keramikvorläufers zu der Emulsion; und
e) Rühren der Emulsion, wenn sich die Partikel innerhalb der Tröpfchen bilden,
wobei der funktionalisierte Keramikvorläufer eine aminofunktionale Gruppe umfasst, die in der Lage ist, das Eindringen der Partikel in Zellen zu fördern.

9. Verfahren nach Anspruch 7 oder 8, wobei die aminofunktionale Gruppe des ersten Keramikvorläufers oder des funktionalisierten Keramikvorläufers in der Lage ist, chemisch, beispielsweise elektrostatisch, mit dem Biomolekül zu interagieren.

10. Verfahren nach einem der Ansprüche 7 bis 9, wobei der erste Keramikvorläufer oder der funktionalisierte Keramikvorläufer ein aminofunktionales Alkoxysilan ist.

11. Verfahren nach Anspruch 7, wobei der erste Keramikvorläufer eine Base ist und die hydrophile Phase einen pH-Wert unter dem pKₐ-Wert des ersten Keramikvorläufers aufweist, wobei das Verfahren die folgenden Schritte umfasst:
Kombinieren der hydrophilen Flüssigkeit und des zweiten Keramikvorläufers;
Einstellen des pH-Werts auf unter den pKₐ-Wert des ersten Keramikvorläufers; und
Hinzufügen des Biomoleküls,
um so die hydrophile Phase zu bilden, wobei die Schritte vor dem Schritt a) durchgeführt werden.

12. Verfahren nach Anspruch 11, wobei der Schritt des Einstellens des pH-Werts Exponieren einer Lösung des zweiten Keramikvorläufers in der hydrophilen Flüssigkeit einem Kationenaustauschharz und dann Trennen der Lösung von dem Harz, sobald der pH-Wert der Lösung einen gewünschten pH-Wert unter dem pKₐ-Wert des ersten Keramikvorläufers erreicht hat, umfasst.

13. Verfahren nach einem der Ansprüche 7 bis 12, wobei das Biomolekül negativ geladen ist oder ausreichend groß ist, dass es nicht in der Lage ist, durch die Poren der Partikel hindurchzudringen.

14. Verfahren nach einem der Ansprüche 7 bis 13, zusätzlich umfassend:
Hinzufügen eines Oberflächenbehandlungsmittels zu der Emulsion nach der Bildung der Partikel, um die Partikel oberflächenzubehandeln.

15. Verfahren nach Anspruch 14, wobei das Oberflächenbehandlungsmittel ein PEG-Silan ist, wie etwa ein Trialkoxysilyl-PEG.

16. Verfahren nach Anspruch 14 oder 15, wobei das Oberflächenbehandlungsmittel eine Zielgruppe zum Abzielen auf ein Ziel in einem Patienten umfasst.

17. Verfahren nach einem der Ansprüche 7 bis 16, wobei ein Polymer oder ein Komplexbildner hinzugefügt ist, sodass es/er innerhalb der Poren der Partikel mit dem Biomolekül angeordnet ist, und wobei das Polymer ein Polyethylinamin, ein Polylysin oder ein Polyhistidin oder eine Substanz, die einen Protonenschwammeffekt bereitstellt, ist.

18. Verfahren nach Anspruch 8, beinhaltend Einstellen des pH-Werts der Emulsion auf mehr als 4, beispielsweise durch Hinzufügung einer Base, wie etwa NaOH, KOH und NH₄OH, vor der Hinzufügung des Biomoleküls und des funktionalisierten Keramikvorläufers.

19. Pharmazeutische Zusammensetzung, umfassend eine partikuläre Substanz nach einem der Ansprüche 1 bis 6, zusammen mit einem pharmazeutisch unbedenklichen Träger, Verdünnungsmittel oder Hilfsstoff.

20. Partikuläre Substanz nach einem der Ansprüche 1 bis 6 zur Verwendung bei der Behandlung einer Erkrankung, Krankheit oder Störung bei einem Säuger.

## Revendications

1. Substance particulaire comprenant :
des particules d'une matrice céramique portant un groupe à fonction amine, le groupe à fonction amine étant capable de promouvoir la pénétration des particules dans des cellules et étant réparti de manière homogène à travers lesdites particules ; et
une biomolécule disposée au sein de pores des particules, la biomolécule pouvant être libérée à partir des particules par dissolution de la matrice céramique.

2. Substance particulaire selon la revendication 1, dans laquelle le groupe fonctionnel interagit chimiquement avec la biomolécule pour prévenir substantiellement la lixiviation.

3. Substance particulaire selon la revendication 1 ou 2, dans laquelle les chaînes polyéthylène glycol sont couplées à la surface des particules.

4. Substance particulaire selon l'une quelconque des revendications 1 à 3, dans laquelle un groupe de ciblage est couplé à la surface des particules.

5. Substance particulaire selon l'une quelconque des revendications 1 à 4, dans laquelle lesdites particules ont une taille moyenne de particules de 20 à 100 nm, et une taille de pores de 1 à 50 nm.

6. Substance particulaire selon l'une quelconque des revendications 1 à 5, dans laquelle un polymère ou un agent complexant est disposé dans les pores des particules avec la biomolécule et fournit un effet d'éponge à protons.

7. Procédé destiné à la fabrication de particules comprenant une biomolécule disposée dans les pores de celles-ci, ledit procédé comprenant :
a) la combinaison :
d'une phase hydrophobe comprenant un liquide hydrophobe, un premier précurseur de céramique et un tensioactif ; et
d'une phase hydrophile comprenant un liquide hydrophile, un deuxième précurseur de céramique et la biomolécule,
de manière à former une émulsion comprenant des gouttelettes de la phase hydrophile dispersées dans la phase hydrophobe ; et
b) l'agitation de l'émulsion pendant que les particules se forment à l'intérieur des gouttelettes ;
dans lequel le premier précurseur de céramique comprend un groupe à fonction amine qui est capable de promouvoir la pénétration des particules dans des cellules.

8. Procédé destiné à la fabrication de particules comprenant une biomolécule disposée dans les pores de celles-ci, ledit procédé comprenant :
a) la combinaison :
d'une phase hydrophobe comprenant un liquide hydrophobe et un tensioactif ; et
d'une phase hydrophile comprenant un liquide hydrophile comprenant de l'eau et un catalyseur comprenant un acide,
de manière à former une émulsion comprenant des gouttelettes de la phase hydrophile dispersées dans la phase hydrophobe ;
b) l'ajout d'un précurseur de céramique à l'émulsion et l'hydrolyse du précurseur de céramique ;
c) l'ajustement du pH de la phase hydrophile jusqu'à une plage adaptée à la biomolécule ;
d) l'ajout de la biomolécule et d'un précurseur de céramique fonctionnalisé à l'émulsion ; et
e) l'agitation de l'émulsion pendant que les particules se forment à l'intérieur des gouttelettes,
dans lequel le précurseur de céramique fonctionnalisé comprend un groupe à fonction amine qui est capable de promouvoir la pénétration des particules dans des cellules.

9. Procédé selon la revendication 7 ou 8, dans lequel le groupe à fonction amine du premier précurseur de céramique ou du précurseur de céramique fonctionnalisé est capable d'interagir chimiquement avec la biomolécule, par exemple d'interagir de manière électrostatique avec celle-ci.

10. Procédé selon l'une quelconque des revendications 7 à 9, dans lequel le premier précurseur de céramique ou le précurseur de céramique fonctionnalisé est un alcoxysilane à fonction amine.

11. Procédé selon la revendication 7, dans lequel le premier précurseur de céramique est une base et la phase hydrophile a un pH au-dessous du pKₐ du premier précurseur de céramique, le procédé comprenant les étapes de :
combinaison du liquide hydrophile et du deuxième précurseur de céramique ;
ajustement du pH jusqu'au-dessous du pKₐ du premier précurseur de céramique ; et
ajout de la biomolécule,
de manière à former la phase hydrophile, lesdites étapes étant conduits avant l'étape a).

12. Procédé selon la revendication 11, dans lequel l'étape d'ajustement du pH comprend l'exposition d'une solution du deuxième précurseur de céramique dans le liquide hydrophile à une résine échangeuse d'ions et puis la séparation de la solution à partir de la résine une fois que le pH de la solution a atteint le pH souhaité au-dessous du pKₐ du premier précurseur de céramique.

13. Procédé selon l'une quelconque des revendications 7 à 12, dans lequel la biomolécule est chargée négativement ou est suffisamment grosse pour ne pas pouvoir passer à travers les pores des particules.

14. Procédé selon l'une quelconque des revendications 7 à 13 comprenant en plus :
l'ajout d'un agent de traitement en surface à l'émulsion suite à la formation des particules de manière à traiter les particules en surface.

15. Procédé selon la revendication 14, dans lequel l'agent de traitement en surface est un PEG-silane, tel qu'un trialcoxysilyle-PEG.

16. Procédé selon la revendication 14 ou 15, dans lequel l'agent de traitement en surface comprend un groupe de ciblage pour cibler une cible chez un patient.

17. Procédé selon l'une quelconque des revendications 7 à 16, dans lequel un polymère ou un agent complexant est ajouté de sorte qu'il est disposé au sein des pores des particules avec la biomolécule, et dans lequel le polymère est une polyéthylinamine, une polylysine, ou une polyhistidine ou une substance qui fournit un effet d'éponge à protons.

18. Procédé selon la revendication 8, incluant l'ajustement du pH de l'émulsion jusqu'à plus de 4, par exemple par l'ajout d'une base, telle que NaOH, KOH et NH₄OH, avant l'ajout de la biomolécule et du précurseur de céramique fonctionnalisé.

19. Composition pharmaceutique comprenant une substance particulaire selon l'une quelconque des revendications 1 à 6, conjointement avec un support, un diluant ou un excipient pharmaceutiquement acceptable.

20. Substance particulaire selon l'une quelconque des revendications 1 à 6, destinée à être utilisée dans le traitement d'une maladie, d'un trouble ou d'une affection chez un mammifère.
